# EUROPEAN PATENT APPLICATION

(11) **EP 4 403 920 A1**
(43) Date of publication of application: **24.07.2024**
(21) Application number: 23212950.2
(22) Date of filing: 29.11.2023
(51) Int. Cl.: G01N 35/00

(54) **SAMPLE ANALYSIS DEVICE**

(30) Priority: 19.01.2023 CN 202310125153
(71) Applicant: Shenzhen New Industries Biomedical Engineering Co., Ltd., Shenzhen, Guangdong 518122 (CN)
(72) Inventor: RAO, Jie, Shenzhen, 518122 (CN); YIN, Li, Shenzhen, 518122 (CN); TANG, Junhui, Shenzhen, 518122 (CN); ZHU, Liang, Shenzhen, 518122 (CN); WANG, Yixian, Shenzhen, 518122 (CN); ZHENG, Xiaolin, Shenzhen, 518122 (CN); HE, Guoyao, Shenzhen, 518122 (CN); XIAO, Hao, Shenzhen, 518122 (CN)
(74) Representative: Studio Torta S.p.A.

(57) **Abstract**

Provided is a sample analysis device, including: a housing (1-10), a reagent preparation apparatus (1.1), a sample processing apparatus (1.2), and an analysis apparatus (1.3). A first independent space (1-21), a second independent space (1-22) and a third independent space (1-23) are sequentially arranged in the housing (1-10) at intervals. The reagent preparation apparatus (1.1) is arranged in the first independent space (1-21). The sample processing apparatus (1.2) is arranged in the second independent space (1-22), a first conveying channel is arranged between the reagent preparation apparatus (1.1) and the sample processing apparatus (1.2), a first switching piece (11-81) is arranged at the first conveying channel, and the first switching piece (11-81) has a first opening state and a first closing state. The analysis apparatus (1.3) is arranged in the third independent space (1-23), a second conveying channel is arranged between the analysis apparatus (1.3) and the sample processing apparatus (1.2), a second switching piece (11-21) is arranged at the second conveying channel, and the second switching piece (11-21) has a second opening state and a second closing state. The technical solution of the present invention effectively solves the problem in a related art of a risk of pollution to a sample, the environment and an operator.

## Description

### Cross Reference to Related Applications

The present invention claims benefit of Chinese Patent Application No. 202310125153.8, filed on January 19, 2023, entitled "Sample Analysis Device", the contents of which are hereby incorporated by reference in its entirety.

### Technical Field

The present invention relates to the technical field of medical instruments, and in particular to a sample analysis device.

### Background

Compared with other arts, molecular diagnosis technology has the advantages of rapidness, high sensitivity, high specificity and the like, and is the most important development and research direction of the in-vitro diagnosis technology. Nucleic acid detection plays a more and more important role as an important application of the molecular diagnosis technology.

The most common nucleic acid detection is a fluorescent quantitative Polymerase Chain Reaction (PCR), which is a process in which a fluorescent dye or a fluorescent marker is used in a nucleic acid amplification process, the intensity of a fluorescence signal is detected by use of an optical apparatus, and a diagnosis result is obtained by processing and analyzing the fluorescence signal.

Traditional PCR detection requires much manual participation and is low in automation degree. Moreover, PCR amplification and detection processes are mostly implemented by different instruments, which need to transfer a sample among different instruments, causing a risk of pollution to the sample, the environment and an operator.

### Summary

The present invention mainly aims to provide a sample analysis device, for solving a problem in the related art of a risk of pollution to a sample, the environment and an operator.

In order to realize the above objective, the present invention provides a sample analysis device, including a housing, a reagent preparation apparatus, a sample processing apparatus and an analysis apparatus. A first independent space, a second independent space and a third independent space are sequentially arranged in the housing at intervals. The reagent preparation apparatus is configured to prepare an extraction reagent and an amplification reagent, and the reagent preparation apparatus is arranged in the first independent space. The sample processing apparatus is configured to extract an analyte from a sample and combine the analyte with the amplification reagent to form a mixture to be detected, the sample processing apparatus is arranged in the second independent space, a first conveying channel is arranged between the reagent preparation apparatus and the sample processing apparatus, a first switching piece is arranged at the first conveying channel, the first switching piece has a first opening state and a first closing state, the first switching piece opens the first conveying channel when in the first opening state, so that the first independent space is communicated with the second independent space, and the first switching piece closes the first conveying channel when in the first closing state, so that the first independent space and the second independent space are independent from each other. The analysis apparatus is configured to analyze the mixture to be detected, the analysis apparatus is arranged in the third independent space, a second conveying channel is arranged between the analysis apparatus and the sample processing apparatus, a second switching piece is arranged at the second conveying channel, the second switching piece has a second opening state and a second closing state, the second switching piece opens the second conveying channel when in the second opening state, so that the second independent space is communicated with the third independent space, and the second switching piece closes the second conveying channel when in the second closing state, so that the second independent space and the third independent space are independent from each other.

In some embodiments, the reagent preparation apparatus further includes a first container and a second container. The first container is configured to place the extraction reagent, and the second container is configured to place the amplification reagent. A first division plate and a second division plate are arranged in the housing at intervals, and the first division plate and the second division plate separate an inner space of the housing to form the first independent space, the second independent space and the third independent space. The first conveying channel is arranged on the first division plate, and the second conveying channel is arranged on the second division plate. The first container and the second container in the first independent space are conveyed into the second independent space by the first conveying channel, and the second container in the second independent space is conveyed into the third independent space by the second conveying channel.

In some embodiments, the sample analysis device further includes a first conveying mechanism arranged in the first independent space or in the second independent space and a second conveying mechanism arranged in the second independent space or in the third independent space. The first conveying mechanism conveys the first container and the second container through the first conveying channel and drives the first switching piece to switch between the first opening state and the first closing state, and the second conveying mechanism conveys the second container through the second conveying channel and drives the second switching piece to switch between the second opening state and the second closing state.

In some embodiments, the first conveying mechanism includes a first rack arranged in the second independent space, a first bearing piece movably arranged on the first rack in a first direction and a first pushing structure arranged at a front end portion of the first bearing piece. The first direction is a direction in which the first independent space, the second independent space and the third independent space are sequentially arranged, the first bearing piece is provided with a first loading position located in the second independent space, and the first bearing piece is provided with a first fetching position in the first independent space, when the first switching piece is pushed to the first opening state by the first pushing structure, the first bearing piece extends into the first independent space and reaches the first fetching position.

In some embodiments, the first pushing structure includes a first rotating piece protruding forwards from the first bearing piece. A rotating axis of the first rotating piece extends in a second direction, and the second direction is perpendicular to the first direction on a horizontal plane. When the first bearing piece moves from the first loading position to the first fetching position, the first rotating piece can push the first switching piece.

In some embodiments, the first division plate includes a first plate body and a second plate body which are arranged at intervals in the first direction. A part of an inner wall of the housing and the first plate body enclose the first independent space, and another part of the inner wall of the housing, the second plate body and the second division plate enclose the second independent space. The first plate body is provided with a first conveying window, the second plate body is provided with a second conveying window corresponding to the first conveying window, and the first conveying window and the second conveying window jointly define the first conveying channel. The first switching piece is pivotally arranged on the first plate body or the second plate body by a first rotating shaft, a first reset member is arranged between the first rotating shaft and the first plate body or the first rotating shaft and the second plate body, and the first reset member keeps the first switching piece in the first closing state under circumstance of applying a first reset force to the first switching piece.

In some embodiments, the second conveying mechanism includes a second rack arranged in the third independent space, a second bearing piece movably arranged on the second rack in the first direction, a second pushing structure arranged at a front end portion of the second bearing piece and an anti-abrasion piece arranged on the second switching piece. The second bearing piece is provided with a second loading position located in the third independent space, and the second bearing piece is provided with a second fetching position in the second independent space, when the second switching piece is pushed to the second opening state by the second pushing structure, the second bearing piece extends into the second independent space and reaches the second fetching position.

In some embodiments, the second pushing structure includes a second rotating piece protruding forwards from the second bearing piece. A rotating axis of the second rotating piece extends in the second direction, and the second direction is perpendicular to the first direction on a horizontal plane. The anti-abrasion piece includes a third rotating piece arranged on the second switching piece. A rotating axis of the third rotating piece is arranged to be parallel to the rotating axis of the second rotating piece, and when the second bearing piece moves from the second loading position to the second fetching position, the second rotating piece can push the second switching piece, and the third rotating piece can be in contact fit with the second bearing piece.

In some embodiments, the second division plate includes a third plate body and a fourth plate body which are arranged at intervals in the first direction. Another part of the inner wall of the housing, the first division plate and the third plate body enclose the second independent space, and the rest part of the inner wall of the housing and the fourth plate body enclose the third independent space. The third plate body is provided with a third conveying window, the fourth plate body is provided with a fourth conveying window corresponding to the third conveying window, and the third conveying window and the fourth conveying window jointly define the second conveying channel. The second switching piece is pivotally arranged on the third plate body or the fourth plate body by a second rotating shaft, a second reset member is arranged between the second rotating shaft and the third plate body or the second rotating shaft and the fourth plate body, and the second reset member keeps the second switching piece in the second closing state under circumstance of applying a second reset force to the second switching piece.

In some embodiments, the first container is a first well plate, and a plurality of first placement holes are formed in the first well plate. The reagent preparation apparatus includes a first rack body and an extraction reagent assembly arranged in the first independent space, and the extraction reagent assembly includes a separation reagent assembly and an enrichment reagent assembly. The first rack body is provided with a reagent preparation platform, and the reagent preparation platform is provided with a first container placement seat for placing the first container. The separation reagent assembly includes a plurality of liquid injection needles movably arranged in the first rack body and located above the reagent preparation platform, each of the plurality of liquid injection needles can be communicated with a corresponding separation reagent, and the plurality of liquid injection needles have a first liquid injection position for moving above the first container placement seat and an avoidance position for being away from the first container. The enrichment reagent assembly includes a pipetting needle and an enrichment reagent bin for bearing an elution buffer and a magnetic bead. The pipetting needle is movably arranged on the first rack body and located above the reagent preparation platform, the enrichment reagent bin is arranged on the reagent preparation platform, and the pipetting needle has a first suction position for moving above the enrichment reagent bin to load the elution buffer and the magnetic bead and a second liquid injection position for moving above the first container placement seat.

In some embodiments, the separation reagent assembly further includes a perfusion rack movably arranged in the first rack body in a vertical direction and located above the reagent preparation platform. The plurality of liquid injection needles are movably arranged on the perfusion rack in the first direction, the first container placement seat is movably arranged on the reagent preparation platform in a second direction, the second direction is perpendicular to the first direction on the horizontal plane, the first container placement seat has a first working position, and when the first container placement seat is in the first working position, the perfusion rack can move in a vertical direction so that the plurality of liquid injection needles are located in the first liquid injection position. The enrichment reagent assembly further includes a reagent arm horizontally and movably arranged on the first rack body and located above the reagent preparation platform. The pipetting needle is movably arranged on the reagent arm in the vertical direction, the first container placement seat further has a second working position located on a side of the first working position, and when the first container placement seat is located in the second working position, the reagent arm can move in the horizontal direction so that the pipetting needle is located in the second liquid injection position.

In some embodiments, the reagent preparation platform is provided with a premix position for placing a premix plate and a second container placement position for placing the second container. The reagent preparation apparatus further includes a preparation reagent bin for placing a first reagent bottle and a second reagent bottle, and the preparation reagent bin is arranged on the reagent preparation platform. The pipetting needle further has a second suction position where the pipetting needle move above the preparation reagent bin to suck the amplification reagent in the preparation reagent bin, a mixing position where the pipetting needle move above the premix plate to fill the amplification reagent to the premix plate and a filling position where the pipetting needle move above the second container to fill a mixed solution in the premix plate to the second container.

In some embodiments, the reagent preparation apparatus further includes a transverse guide rail and a longitudinal guide rail. The transverse guide rail and the longitudinal guide rail are both located above the reagent preparation platform. The longitudinal guide rail is arranged on the first rack body and extends in the first direction, the transverse guide rail extends in a second direction perpendicular to the first direction on a horizontal plane, the transverse guide rail is movably arranged below the longitudinal guide rail in an extending direction of the longitudinal guide rail, the reagent arm is movably arranged on the transverse guide rail in an extending direction of the transverse guide rail, and the pipetting needle is movably arranged on the reagent arm in the vertical direction.

In some embodiments, the second container is a second well plate, the second well plate is provided with a plurality of second placement holes, and a number of the first placement holes is greater than a number of the second placement holes.

In some embodiments, the sample processing apparatus includes: a sample transfer mechanism, a filling mechanism, and an extraction mechanism. The sample transfer mechanism includes a transfer rack movable in a second direction. The transfer rack has a first placement position, and the transfer rack has a transfer position and a sample loading position in a moving direction of the transfer rack. The filling mechanism includes a sample arm, and when the transfer rack moves to the sample loading position, the sample arm moves to the sample loading position and fills a sample or sucks the analyte into the first container in the first placement position. The extraction mechanism includes an analyte extraction piece and an extraction rack. The extraction rack is movably arranged in the second direction and has a second placement position, and the extraction rack has a placement position and an extraction position in a moving direction of the extraction rack. When the extraction rack moves to the placement position and the transfer rack moves to the transfer position, the first container is moved from the first placement position to the second placement position, and when the extraction rack moves to the extraction position, the analyte extraction piece can extract the analyte from the first container in the second placement position.

In some embodiments, the sample processing apparatus further includes a storage mechanism and a transmission mechanism. The storage mechanism includes a sample storage bin for placing a sample tube and a quality control substance storage bin for placing a quality control tube that contains a quality control substance. The filling mechanism further includes a consumable loading mechanism for storing a consumable, the sample arm can fill the sample in the sample tube or the quality control substance in the quality control tube into the first container, the sample arm is provided with a sample loading needle, and the sample loading needle can be connected with the consumable in a fitting manner. The transmission mechanism includes a moving rack which is movably arranged. The moving rack is provided with a bearing position for placing the sample tube or the quality control tube, the moving rack has a storage transmission position arranged corresponding to the storage mechanism and a filling transmission position arranged corresponding to the filling mechanism, and the moving rack can move between the storage transmission position and the filling transmission position. The transport mechanism includes a grabbing piece capable of transporting the sample tube between the moving rack and the sample storage bin and transporting the quality control tube between the moving rack and the quality control substance storage bin, and the grabbing piece can grab the sample tube from the sample storage bin or grab the quality control tube from the quality control substance storage bin.

In some embodiments, the sample analysis device further includes a sample processing gripper, and the sample processing gripper is provided with a second gripper structure. The sample processing apparatus further includes a film sealing mechanism arranged in the second independent space, and the film sealing mechanism includes: a third rack, a hot-pressing mechanism, and a bearing mechanism. The hot-pressing mechanism is movably arranged on the third rack in a vertical direction, and the second gripper structure is arranged on a side of the hot-pressing mechanism. The bearing mechanism includes a first placement area and a second placement area which are adjacently arranged, and the bearing mechanism is movably arranged between the hot-pressing mechanism and the second gripper structure, so that the bearing mechanism having a hot-pressing position for fitting the first placement area and the hot-pressing mechanism, a clamping position for fitting the second placement area and the second gripper structure, and an assembly position for fitting the first placement area and the second gripper structure. The first placement area is configured to place the second container, the second container is configured to place the mixture to be detected, and the second placement area is configured to place a cover body.

In some embodiments, the analysis apparatus includes a plurality of amplification mechanisms sequentially arranged in a second direction. Each of the plurality of amplification mechanisms includes: a second rack body, a detection apparatus, a reagent loading apparatus, and a temperature control assembly. The second rack body is provided with a detection window. The detection apparatus is arranged on the second rack body and located at the detection window. The reagent loading apparatus includes a bracket. The second container is detachably arranged on the bracket, the bracket is movably arranged on the second rack body in the first direction, and the second direction is perpendicular to the first direction on a horizontal plane. The second container moves along with the bracket and has a storage position located below the detection window and a loading position for moving out of the second rack body. The temperature control assembly is movably arranged on the second rack body in a vertical direction and can drive the second container to ascend and descend, so that the second container to be lifted to a detection position in butt joint fit with the detection apparatus.

In some embodiments, the sample analysis device further includes a reagent preparation gripper, a sample processing gripper, and an analysis gripper. The reagent preparation gripper can move in the first direction and/or a second direction in the first independent space, and the second direction is perpendicular to the first direction on a horizontal plane. The reagent preparation gripper has a first gripper structure capable of moving in a vertical direction, and the first gripper structure is configured to grip the first container or the second container located in the first independent space. The sample processing gripper can move in the first direction in the second independent space. The sample processing gripper has a second gripper structure capable of moving in the vertical direction, and the second gripper structure is configured to grip the first container or the second container located in the second independent space. The analysis gripper can move in the second direction in the third independent space, the analysis gripper has a third gripper structure capable of moving in the vertical direction, and the third gripper structure is configured to grip the second container located in the third independent space.

By applying the technical solution of the present invention, the sample analysis device includes: the housing, the reagent preparation apparatus, the sample processing apparatus, and the analysis apparatus. The first independent space, the second independent space and the third independent space are sequentially arranged in the housing at intervals. The reagent preparation apparatus is configured to prepare the extraction reagent and the amplification reagent. The reagent preparation apparatus is arranged in the first independent space. The sample processing apparatus is configured to extract the analyte from the sample and combine the analyte with the amplification reagent to form the mixture to be detected. The sample processing apparatus is arranged in the second independent space. The first conveying channel is arranged between the reagent preparation apparatus and the sample processing apparatus. The first switching piece is arranged at the first conveying channel, and the first switching piece has a first opening state and a first closing state. The first switching piece opens the first conveying channel when in the first opening state, so that the first independent space is communicated with the second independent space, and the first switching piece closes the first conveying channel when in the first closing state, so that the first independent space and the second independent space are independent from each other. The analysis apparatus is configured to analyze the mixture to be detected. The analysis apparatus is arranged in the third independent space. The second conveying channel is arranged between the analysis apparatus and the sample processing apparatus. The second switching piece is arranged at the second conveying channel, and the second switching piece has a second opening state and a second closing state. The second switching piece opens the second conveying channel when in the second opening state, so that the second independent space is communicated with the third independent space, and the second switching piece closes the second conveying channel when in the second closing state, so that the second independent space and the third independent space are independent from each other.

When the first switching piece is in the first closing state and the second switching piece is in the second closing state, the first independent space, the second independent space, and the third independent space are all independent from each other. At this moment, the extraction reagent and the amplification reagent are prepared in the first independent space, and the sample located in the second independent space cannot enter the first independent space and cannot be transferred to the outside of the sample analysis device, thereby avoiding pollution to the environment and the operator. Meanwhile, an external potential pollution source likewise cannot enter the sample analysis device, thereby avoiding affecting the accuracy of detection. In the process of conveying the prepared extraction reagent and the prepared amplification reagent into the second independent space through the first conveying channel, the first switching piece is in the first opening state, and after the extraction reagent and the amplification reagent are conveyed into the second independent space, the first switching piece is in the first closing state again. At this moment, the sample processing apparatus located in the second independent space processes the sample, places the sample in the extraction reagent, extracts the analyte from the sample, and combines the analyte with the amplification reagent to form the mixture to be detected. In the process of conveying the mixture to be detected into the third independent space through the second conveying channel, the second switching piece is in the second opening state, and after the mixture to be detected is conveyed into the third independent space, the second switching piece is in the second closing state again. At this moment, the analysis apparatus located in the third independent space analyzes the mixture to be detected. Thus, preparing the extraction reagent and the amplification reagent, then placing the sample in the extraction reagent to extract the analyte from the sample, forming the mixture to be detected, and analyzing the mixture to be detected are all implemented in the housing. The sample does not need to be transferred between different instruments in the related art, and only needs to be transferred in one direction inside the housing, so that the risk of pollution to the sample, the environment and the operator can be reduced. Meanwhile, it is ensured that there is no need to generate additional interaction with the outside of the sample analysis device during detection, thereby ensuring the accuracy of detection. Moreover, when the first independent space, the second independent space and the third independent space work in respective independent spaces, the adjacent independent spaces are isolated through the first switching piece and/or the second switching piece to prevent cross pollution between different spaces. Only when the extraction reagent and the amplification reagent or the mixture to be detected needs to be conveyed, the first switching piece and/or the second switching piece can be opened. When the extraction reagent and the amplification reagent or the mixture to be detected does not need to be conveyed, the three independent spaces are mutually independent and closed, pollution cannot be transmitted between the three independent spaces, and the risk of pollution to the sample, the environment and the operator can be reduced. Therefore, the technical solution of the present invention can solve the problem in the related art of the risk of pollution to the sample, the environment and the operator.

### Brief Description of the Drawings

The drawings consisting of a part of the invention are used for further understanding of the present invention. The schematic embodiments and description thereof are used for explaining the invention and do not limit the invention improperly. In the drawings,
Fig. 1 illustrates a schematic three-dimensional structural diagram of a housing according to an embodiment of a sample analysis device of the present invention;
Fig. 2 illustrates a schematic three-dimensional structural diagram of main view angles of a reagent preparation apparatus, a sample processing apparatus, and an analysis apparatus of the sample analysis device of Fig. 1;
Fig. 3 illustrates a partial enlarged schematic diagram at parts A of the reagent preparation apparatus, the sample processing apparatus, and the analysis apparatus of Fig. 2;
Fig. 4 illustrates a partial enlarged schematic diagram at parts B of the reagent preparation apparatus, the sample processing apparatus, and the analysis apparatus of Fig. 2;
Fig. 5 illustrates a schematic three-dimensional structural diagram of rear view angles of the reagent preparation apparatus, the sample processing apparatus, and the analysis apparatus of the sample analysis device of Fig. 1;
Fig. 6 illustrates a partial enlarged schematic diagram at parts C of the reagent preparation apparatus, the sample processing apparatus, and the analysis apparatus of Fig. 5;
Fig. 7 illustrates a partial schematic diagram of a second conveying mechanism of the sample analysis device of Fig. 1 passing through a second conveying channel;
Fig. 8 illustrates a top view of the reagent dispensing apparatus, the sample processing apparatus, and the analysis apparatus of Fig. 2;
Fig. 9 illustrates a schematic three-dimensional structural diagram of a first conveying mechanism of the sample analysis device of Fig. 2 bearing a first container;
Fig. 10 illustrates a schematic three-dimensional structural diagram of a first conveying mechanism of the sample analysis device of Fig. 2 bearing a second container;
Fig. 11 illustrates a partial side view of a first bearing piece of the sample analysis device of Fig. 1 driving a first switching piece to open a first conveying channel;
Fig. 12 illustrates a partial schematic three-dimensional structural diagram of a second bearing piece of the sample analysis device of Fig. 1 driving a second switching piece to open a second conveying channel;
Fig. 13 illustrates a schematic three-dimensional structural diagram of a first viewing angle of a reagent preparation apparatus of the sample analysis device of Fig. 1;
Fig. 14 illustrates a schematic three-dimensional structural diagram of a second viewing angle of a reagent preparation apparatus of the sample analysis device of Fig. 1;
Fig. 15 illustrates a schematic three-dimensional structural diagram of a reagent arm of the reagent preparation apparatus of Fig. 14;
Fig. 16 illustrates a schematic three-dimensional structural diagram of a separation reagent assembly of the reagent preparation apparatus of Fig. 14;
Fig. 17 illustrates a top view of a sample processing device of the sample analysis device of Fig. 1;
Fig. 18 illustrates a schematic three-dimensional structural diagram of a transport mechanism of the sample processing apparatus of Fig. 17;
Fig. 19 illustrates a schematic three-dimensional structural diagram of a reagent arm and a sample loading needle of the sample processing apparatus of Fig. 17;
Fig. 20 illustrates a schematic three-dimensional structural diagram of a film sealing mechanism of the sample processing apparatus of Fig. 17;
Fig. 21 illustrates a schematic three-dimensional structural diagram of an amplification mechanism of an analysis apparatus of the sample analysis device of Fig. 1; and
Fig. 22 illustrates a schematic three-dimensional structural diagram of a frame assembly and a temperature control assembly of the amplification mechanism of Fig. 21.

Herein, the above drawings include the following reference numbers:
1-10: housing; 1-11: first division plate; 1-111 : first plate body; 1-131: first conveying window; 1-112: second plate body; 1-132: second conveying window; 1-12: second division plate; 1-121: third plate body; 1-133: third conveying window; 1-122: fourth plate body; 1-134: fourth conveying window; 1-21: first independent space; 1-22: second independent space; 1-23: third independent space;
1.1: reagent preparation apparatus; 11 -20: first container; 10-21: first placement hole group; 11-211: first placement hole; 11-30: second container; 11-31: second placement hole; 11-81: first switching piece; 11-83: first reset member; 11-21: second switching piece; 11-22: second reset member; 11-40: cover body; 10-10: first rack body; 10-11: reagent preparation platform; 10-111: premix position: 10-112: second container placement position: 10-22: preparation reagent bin: 10-30: separation reagent assembly: 10-31: liquid injection needle: 10-32: perfusion rack; 10-40: enrichment reagent assembly; 10-41: pipetting needle; 10-42: enrichment reagent bin; 10-44: reagent arm; 2-40: transverse guide rail; and 2-41: longitudinal guide rail;
1.2: sample processing apparatus; 31-10: sample transfer mechanism; 31-11: transfer rack; 31-13: sample arm; 31-14: sample loading needle; 31- 21: analyte extraction piece; 31-22: extraction rack; 31-30: transmission mechanism; 31-31: moving rack; 31-311: bearing position; 31-40: transport mechanism; 31-411: grabbing piece; 32-10: storage mechanism; 32-11: sample storage bin; 32-12: quality control substance storage bin; 32-123: bin door; 32-20: filling mechanism; 32-21: consumable loading mechanism; 34-30: waste liquid recovery mechanism; 34-311: recovery position; 33-30: film sealing mechanism; 33-10: third rack; 33-20: hot-pressing mechanism; 33-40: bearing mechanism;
1.3: analysis apparatus; 4-01: amplification mechanism: 4-100: second rack body: 4-10: outer frame: 4-20: inner frame: 4-30: lifting frame member: 4-33: bearing beam: 4-200: detection apparatus; 4-110: detection window: 4-300: reagent loading apparatus; 4-310: bracket; 4-410: temperature control assembly; 4-210: heat cover; 4-420: driving assembly;
5-01: first conveying mechanism; 5-10: first rack; 5-20: first bearing piece; 5-30: first pushing structure; 5-31: first rotating piece; 6-01: second conveying mechanism; 6-31: second rack; 6-32: second bearing piece; 6-322: second pushing structure; 6-40: third rotating piece; 6-3221: second rotating piece;
7-50: reagent preparation gripper; 7-52: first gripper structure; 7-20: sample processing gripper; 7-21: second gripper structure; 7-30: analysis gripper; and 7-31: third gripper structure.

### Detailed Description of the Embodiments

The technical solutions in the embodiments of the present invention will be clearly and completely described below in combination with the drawings in the embodiments of the present invention. It is apparent that the described embodiments are not all embodiments but part of embodiments of the present invention. In fact, the description of at least one exemplary embodiment below is merely illustrative, and will not be taken as any limitation to the present invention and its application or use. All other embodiments obtained by those of ordinary skill in the art on the basis of the embodiments in the invention without creative work shall fall within the scope of protection of the present invention.

It should be noted that the terms used here are only for describing specific implementation modes, and are not intended to limit the exemplary implementation modes according to the application. As used herein, the singular form is also intended to include the plural form unless the context clearly indicates otherwise. In addition, it should also be understood that when the terms "comprising" and/or "including" are used in this specification, same indicate the presence of features, steps, operations, devices, components and/or combinations thereof.

Unless otherwise specified, relative arrangements of parts and steps, digital expressions and values elaborated in these embodiments will not limit the scope of the invention. Meanwhile, it should be understood, to facilitate the description, that the sizes of all parts shown in the drawings are not drawn according to an actual proportionate relationship. The technologies, the methods and the equipment known by those of ordinary skill in the art may not be elaborated. However, where appropriate, the technologies, the methods and the equipment should be deemed to be part of the authorized specification. In all examples shown and discussed here, any specific values should be merely explained to be exemplary rather than taken as a limitation. Therefore, other examples of the exemplary embodiments may have different values. It should be noted that the similar reference numbers and letters indicate the similar items in the drawings below, so there is no need to further discuss it in subsequent drawings once an item is defined in one drawing.

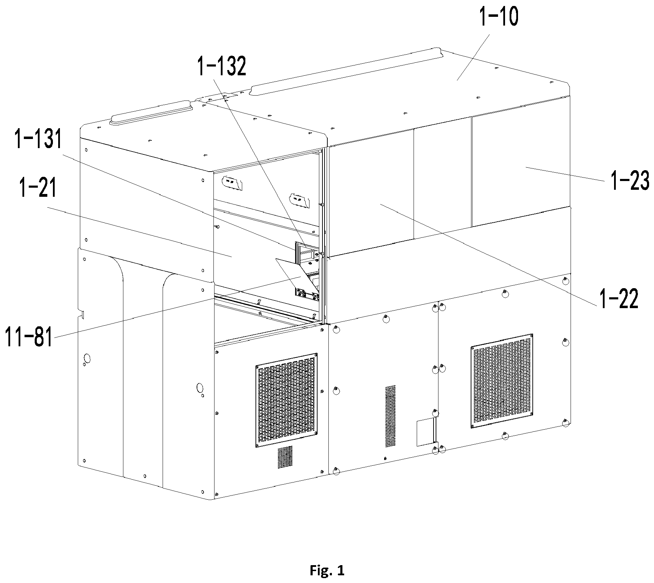

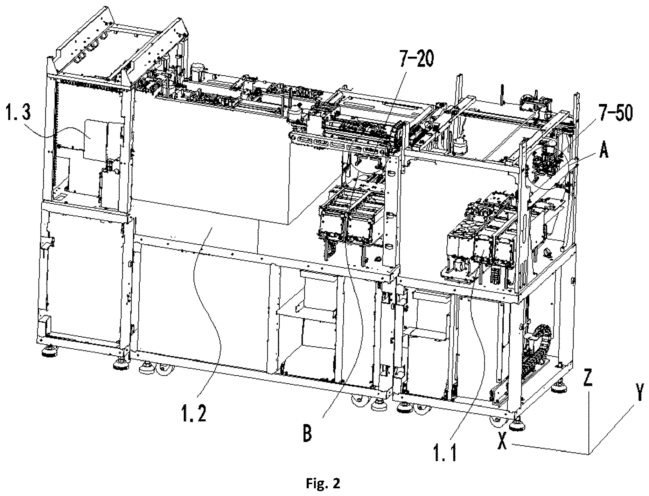

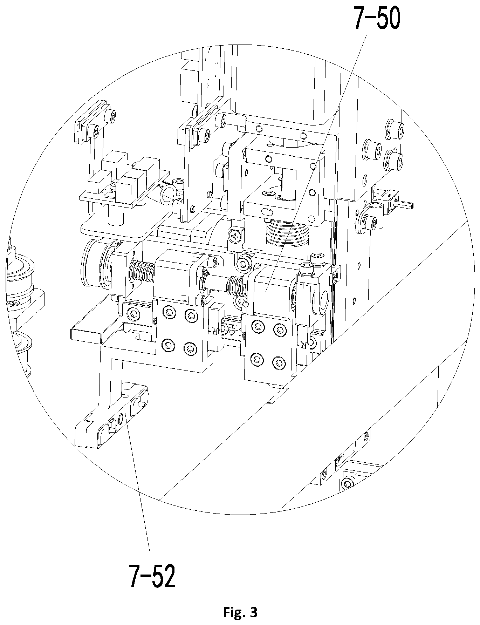

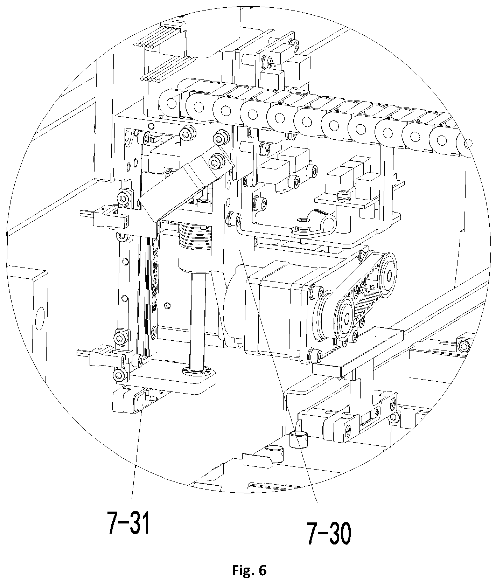

As shown in Figs. 1 to 8, the sample analysis device in the present embodiment includes: a housing 1-10, a reagent preparation apparatus 1.1, a sample processing apparatus 1.2 and an analysis apparatus 1.3. A first independent space 1-21, a second independent space 1-22 and a third independent space 1-23 are sequentially arranged in the housing 1-1 at intervals. The reagent preparation apparatus 1.1 is configured to prepare an extraction reagent and an amplification reagent. The reagent preparation apparatus 1.1 is arranged in the first independent space 1-21. The sample processing apparatus 1.2 is configured to extract an analyte from a sample and combine the analyte with the amplification reagent to form a mixture to be detected. The sample processing apparatus 1.2 is arranged in the second independent space 1-22. A first conveying channel is arranged between the reagent preparation apparatus 1.1 and the sample processing apparatus 1.2. A first switching piece 11-81 is arranged at the first conveying channel, and the first switching piece 11-81 has a first opening state and a first closing state. The first switching piece 11-81 opens the first conveying channel when in the first opening state, so that the first independent space 1-21 is communicated with the second independent space 1-22, and the first switching piece 11-81 closes the first conveying channel when in the first closing state, so that the first independent space 1-21 and the second independent space 1-22 are independent from each other. The analysis apparatus 1.3 is configured to analyze the mixture to be detected. The analysis apparatus 1.3 is arranged in the third independent space 1-23. The second conveying channel is arranged between the analysis apparatus 1.3 and the sample processing apparatus 1.2. The second switching piece 11-21 is arranged at the second conveying channel, and the second switching piece 11-21 has a second opening state and a second closing state. The second switching piece 11-21 opens the second conveying channel when in the second opening state, so that the second independent space 1-22 is communicated with the third independent space 1-23, and the second switching piece 11-21 closes the second conveying channel when in the second closing state, so that the second independent space 1-22 and the third independent space 1-23 are independent from each other.

By applying the technical solution of the present embodiment, the reagent preparation apparatus 1.1 is configured to prepare the extraction reagent and the amplification reagent. The reagent preparation apparatus 1.1 is arranged in the first independent space 1-21. The sample processing apparatus 1.2 is configured to extract the analyte from the sample and combine the analyte with the amplification reagent to form the mixture to be detected. The sample processing apparatus 1.2 is arranged in the second independent space 1-22. The analysis apparatus 1.3 is configured to analyze the mixture to be detected. The analysis apparatus 1.3 is arranged in the third independent space 1-23. When the first switching piece 11-81 is in the first closing state and the second switching piece 11-21 is in the second closing state, the first independent space 1-21, the second independent space 1-22, and the third independent space 1-23 are all independent from each other. At this moment, the extraction reagent and the amplification reagent are prepared in the first independent space 1-21, and the sample located in the second independent space 1-22 cannot enter the first independent space 1-21 and cannot be transferred to the outside of the sample analysis device, thereby avoiding pollution to the environment and the operator. Meanwhile, an external potential pollution source likewise cannot enter the sample analysis device, thereby avoiding affecting the accuracy of detection. In the process of conveying the prepared extraction reagent and the prepared amplification reagent into the second independent space 1-22 through the first conveying channel, the first switching piece 11-81 is in the first opening state, and after the extraction reagent and the amplification reagent are conveyed into the second independent space 1-22, the first switching piece 11-81 is in the first closing state again. At this moment, the sample processing apparatus 1.2 located in the second independent space 1-22 processes the sample, places the sample in the extraction reagent, extracts the analyte from the sample, and combines the analyte with the amplification reagent to form the mixture to be detected. In the process of conveying the mixture to be detected into the third independent space 1-23 through the second conveying channel, the second switching piece 11-21 is in the second opening state, and after the mixture to be detected is conveyed into the third independent space 1-23, the second switching piece 11-21 is in the second closing state again. At this moment, the analysis apparatus 1.3 located in the third independent space 1-23 analyzes the mixture to be detected. Thus, preparing the extraction reagent and the amplification reagent, then placing the sample in the extraction reagent to extract the analyte from the sample, forming the mixture to be detected, and analyzing the mixture to be detected are all implemented in the housing 1-10. The sample does not need to be transferred between different instruments in the related art, and only needs to be transferred in one direction inside the housing 1-10, so that the risk of pollution to the sample, the environment and the operator can be reduced. Meanwhile, it is ensured that there is no need to generate additional interaction with the outside of the sample analysis device during detection, thereby ensuring the accuracy of detection. Moreover, when the first independent space 1-21, the second independent space 1-22 and the third independent space 1-23 work in respective independent spaces, the adjacent independent spaces are isolated through the first switching piece 11-81 and/or the second switching piece 11-21 to prevent cross pollution between different spaces. Only when the extraction reagent and the amplification reagent or the mixture to be detected needs to be conveyed, the first switching piece 11-81 and/or the second switching piece 11-21 can be opened. When the extraction reagent and the amplification reagent or the mixture to be detected does not need to be conveyed, the three independent spaces are mutually independent and closed, pollution cannot be transmitted between the three independent spaces, and the risk of pollution to the sample, the environment and the operator can be reduced. Therefore, the technical solution of the present invention can solve the problem in the related art of the risk of pollution to the sample, the environment and the operator.

In the present embodiment, the above being transferred in one direction in the housing 1-10 means that the first container 11-20 and the second container 11-30 are transferred from the first independent space 1-21 into the second independent space 1-22, and the second container 11-30 is transferred from the second independent space 1-22 into the third independent space 1-23 without reverse transfer. The first switching piece 11-81 is preferably a first opening and closing door, and the second switching piece 11-21 is preferably a second opening and closing door.

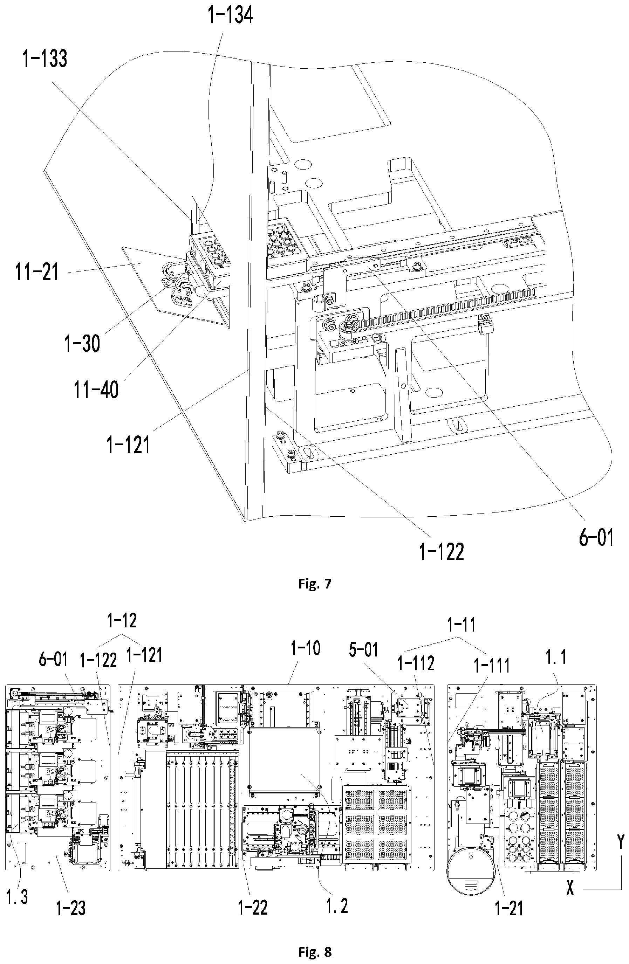

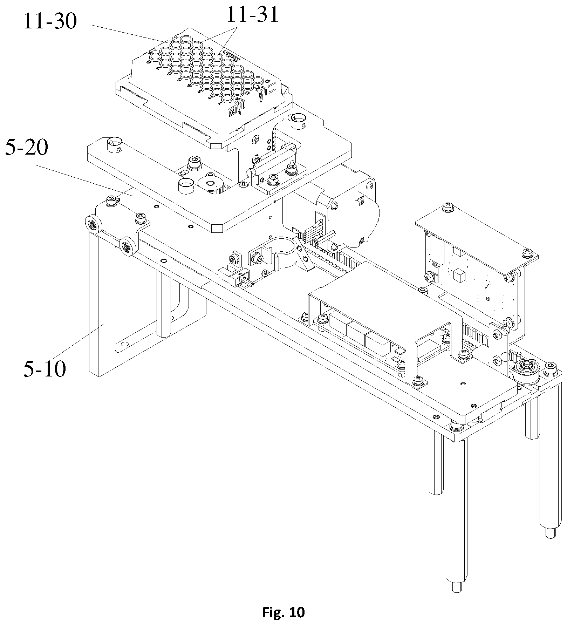

As shown in Figs. 7 to 10, the reagent preparation apparatus 1.1 further includes a first container 11-20 and a second container 11-30. The first container 11-20 is configured to place the extraction reagent, and the second container 11-30 is configured to place the amplification reagent. In order to cause the first independent space 1-21, the second independent space 1-22 and the third independent space 1-23 mutually independent and closed, a first division plate 1-11 and a second division plate 1-12 are arranged in the housing 1-10 at intervals, and the first division plate 1-11 and the second division plate 1-12 separate the inner space of the housing 1-10 to form the first independent space 1-21, the second independent space 1-22 and the third independent space 1-23. In order to facilitate conveying the first container 11-20 and the second container 11-30 in the first independent space 1-21 to the second independent space 1-22 and in order to facilitate conveying the second container 11-30 in the second independent space 1-22 to the third independent space 1-23, the first conveying channel is arranged on the first division plate 1-11, and the second conveying channel is arranged on the second division plate 1-12. The first container 11-20 and the second container 11-30 in the first independent space 1-21 are conveyed into the second independent space 1-22 by means of the first conveying channel, and the second container 11-30 in the second independent space 1-22 is conveyed into the third independent space 1-23 by means of the second conveying channel.

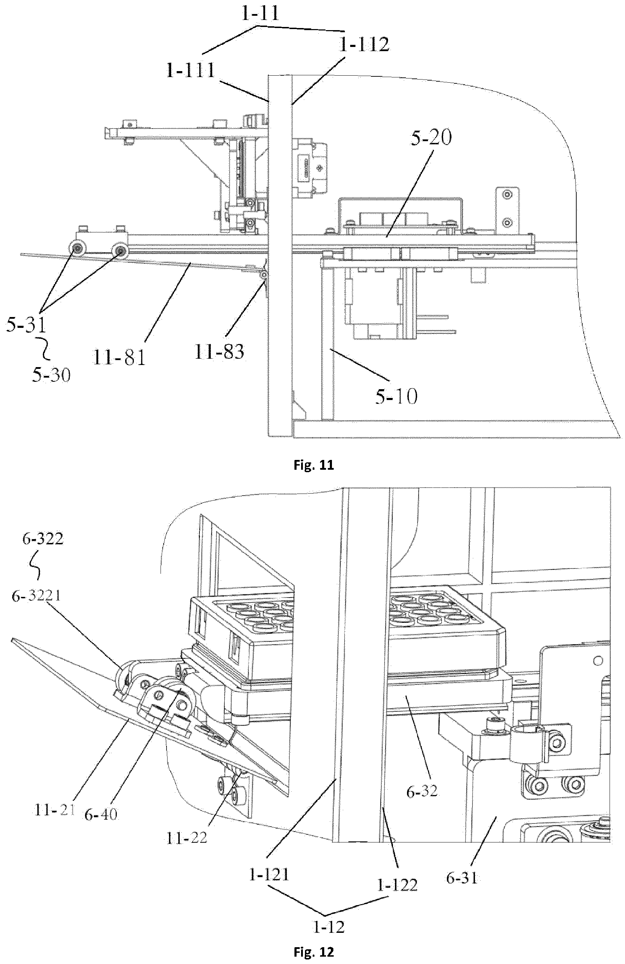

As shown in Figs. 8 to 11, the sample analysis device further includes a first conveying mechanism 5-01 arranged in the second independent space 1-22 and a second conveying mechanism 6-01 arranged in the third independent space 1-23. The first conveying mechanism 5-01 conveys the first container 11-20 and the second container 11-30 through the first conveying channel and drives the first switching piece 11-81 to switch between the first opening state and the first closing state, and the second conveying mechanism 6-01 conveys the second container 11-30 through the second conveying channel and drives the second switching piece 11-21 to switch between the second opening state and the second closing state. Thus, since the first conveying mechanism 5-01 specially conveying the first container 11-20 and the second container 11-30 is arranged, the first container 11-20 and the second container 11-30 can smoothly and conveniently pass through the first conveying channel, and meanwhile the first switching piece 11-81 is further driven to open or close, to automatically perform opening or closing operation, without the need of additionally arranging a driving mechanism for driving the first switching piece 11-81 to open or close, so that the first switching piece 11-81 is switched between the first opening state and the first closing state more quickly, reducing the possibility that the sample located in the second independent space 1-22 is polluted into the first independent space 1-21, and ensuring the effect of isolation to avoid pollution by means of the first switching piece 11-81. Similarly, the second conveying mechanism 6-01 for conveying the second container 11-30 and the second switching piece 11-21 are in driving fit to achieve the same technical effect.

Certainly, in some other embodiments, the sample analysis device further includes a first conveying mechanism arranged in the first independent space and a second conveying mechanism arranged in the second independent space.

As shown in Figs. 1 to 10, in order to facilitate loading of the first container 11-20 or the second container 11-30 onto the first conveying mechanism 5-01 or unloading of the first container 11-20 or the second container 11-30 from the first conveying mechanism 5-01, and in order to facilitate loading of the second container 11-30 onto the second conveying mechanism 6-01 or unloading of the second container 11-30 from the second conveying mechanism 6-01. The sample analysis device further includes a reagent preparation gripper 7-50, a sample processing gripper 7-20 and an analysis gripper 7-30. The reagent preparation gripper 7-50 can move in the first direction and/or the second direction in the first independent space 1-21, and the second direction is perpendicular to the first direction in the horizontal plane. The reagent preparation gripper 7-50 has a first gripper structure 7-52 capable of moving in the vertical direction, and the first gripper structure 7-52 is configured to grip the first container 11-20 or the second container 11-30 located in the first independent space 1-21. The sample processing gripper 7-20 can move in the first direction in the second independent space 1-22. The sample processing gripper 7-20 has a second gripper structure 7-21 capable of moving in the vertical direction, and the second gripper structure 7-21 is configured to grip the first container 11-20 or the second container 11-30 located in the second independent space 1-22. Thus, the first gripper structure 7-52 respectively grabs the first container 11-20 and the second container 11-30 to the first conveying mechanism 5-01, and the first container 11-20 and the second container 11-30 are conveyed across the first independent space 1-21 and the second independent space 1-22 by means of the first conveying mechanism 5-01, so that the first container 11-20 which is located in the first independent space 1-21 and on which the extraction reagent is placed and the second container 11-30 on which the amplification reagent is placed can be conveyed into the second independent space 1-22. The second gripper structure 7-21 can also grab away the first container 11-20 or the second container 11-30 on the first conveying mechanism 5-01 located in the second independent space 1-22. After the sample processing apparatus 1.2 processes the sample in the first container 11-20 to form the mixture to be detected, the second gripper structure 7-21 grabs the second container 11-30 located in the second independent space 1-22 onto the second conveying mechanism 6-01, and the second container 11-30 is conveyed across the second independent space 1-22 and the third independent space 1-23 by means of the second conveying mechanism 6-01, so that the second container 11-30 which is located in the second independent space 1-22 and on which the mixture to be detected is placed can be conveyed into the third independent space 1-23. The analysis gripper 7-30 can move in the second direction in the third independent space 1-23, the analysis gripper 7-30 has a third gripper structure 7-31 capable of moving in the vertical direction, and the third gripper structure 7-31 is configured to grip the second container 11-30 located in the third independent space 1-23. The third gripper structure 7-31 grabs away the second container 11-30 on the second conveying mechanism 6-01 located in the third independent space 1-23. Thus, the first container 11-20 can be transferred in the first independent space 1-21 and the second independent space 1-22, and the second container 11-30 can be transferred in the first independent space 1-21, the second independent space 1-22 and the third independent space 1-23, thereby realizing automatic transfer of the first container 11-20 and the second container 11-30 of the sample analysis device.

It is to be noted that the first direction in the present embodiment refers to an X-axis direction in the horizontal plane, the second direction refers to a Y-axis direction in the horizontal plane, and the vertical direction is a Z-axis direction. Certainly, in some other embodiments, the first direction may refer to the Y-axis direction in the horizontal plane, and the second direction may refer to the X-axis direction in the horizontal plane.

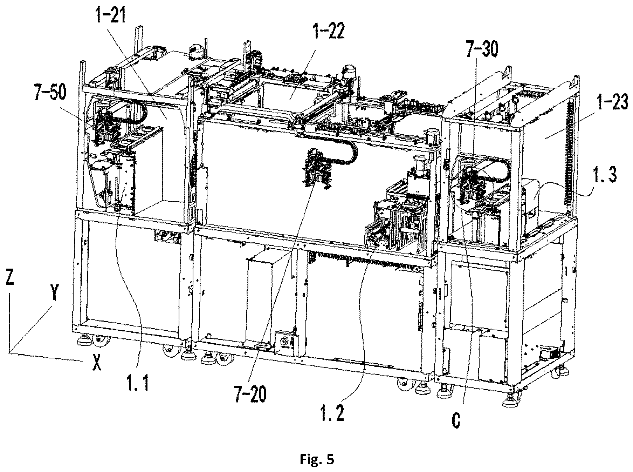

As shown in Fig. 5 and Figs. 8 to 11, the first conveying mechanism 5-01 includes a first rack 5-10 arranged in the second independent space 1-22, a first bearing piece 5-20 movably arranged on the first rack 5-10 in the first direction and a first pushing structure 5-30 arranged at a front end portion of the first bearing piece 5-20. The first direction is a direction in which the first independent space 1-21, the second independent space 1-22 and the third independent space 1-23 are sequentially arranged, the first bearing piece 5-20 is provided with a first loading position located in the second independent space 1-22, and the first bearing piece 5-20 is provided with a first fetching position in the first independent space 1-21 ,when the first switching piece 11-81 is pushed to the first opening state by means of the first pushing structure 5-30, the first bearing piece 5-20 extends into the first independent space 1-21 and reaches the first fetching position. Thus, the first container 11-20 or the second container 11-30 can be transported between different positions. Compared with a gripper structure which is complex in structure, high in cost and high in space requirement and needs to reserve a larger space, the above structure is simpler in structure and lower in production cost. Meanwhile, since the structure of the first conveying mechanism 5-01 is simple, the failure rate of the first conveying mechanism 5-01 can be effectively reduced, thereby ensuring the stability of the first conveying mechanism 5-01 during operation.

It is to be noted that the front end portion of the first bearing piece 5-20 refers to an end portion, facing the first independent space, of the first bearing piece 5-20 in a reverse direction of the X axis.

After long-term research, it is found by the inventor that, within the housing 1-10, the more samples in the independent space, the higher the pollution possibility of the samples is. Specifically, the first independent space 1-21, the second independent space 1-22, and the third independent space 1-23 are divided in the housing 1-10 according to the first direction. The reagent preparation apparatus 1.1 is configured to prepare the extraction reagent and the amplification reagent in the first independent space 1-21, and the first independent space 1-21 does not contain the sample and belongs to a low-pollution area. The sample processing apparatus 1.2 extracts the analyte from the sample within the second independent space 1-22 and combines the analyte with the amplification reagent to form the mixture to be detected. The second independent space 1-22 contains the sample, but the content of the sample is low, so the second independent space belongs to a medium-pollution area. The analysis apparatus 1.3 analyzes the mixture to be detected in the third independent space 1-23. The content of the sample in the third independent space 1-23 is greater than the content of the sample in the second independent space 1-22, so the third independent space belongs to a high-pollution area. In order to reduce the risk that the space with a low pollution level is polluted by the space with a high pollution level, the present embodiment designs the first independent space 1-21, the second independent space 1-22 and the third independent space 1-23, and the division plate is arranged between two adjacent independent spaces to form a physical isolation between the two adjacent independent spaces. However, the arrangement of the division plate may cause obstacles for moving the sample or reagent to another space. In order to solve this problem, in the sample analysis device of the present embodiment, the first switching piece 11-81 is arranged on the first division plate 1-11, the second switching piece 11-21 is arranged on the second division plate 1-12, and then the first conveying mechanism 5-01 is designed to push the first switching piece 11-81 to achieve cross-space transportation of the reagent. Similarly, the second conveying mechanism 6-01 is also designed to push the second switching piece 11-21 to achieve cross-space transportation of the sample. Specifically,

as shown in Figs. 8 to 11, the first pushing structure 5-30 includes a first rotating piece 5-31 protruding forwards from the first bearing piece 5-20. A rotating axis of the first rotating piece 5-31 extends in the second direction, and the second direction is perpendicular to the first direction in the horizontal plane. When the first bearing piece 5-20 moves from the first loading position to the first fetching position, the first rotating piece 5-31 can push the first switching piece 11-81.

In the above structure, when the first conveying mechanism 5-01 is mounted, the first rack 5-10 may be fixed in the first independent space 1-21 or the second independent space 1-22. Since the first bearing piece 5-20 can move in the first direction and has the first loading position and the first fetching position, the first bearing piece 5-20 can realize cross-space transportation of the sample or reagent during activity. Specifically, when the first bearing piece 5-20 is located in the first loading position, the first rack 5-10 and the first bearing piece 5-20 are in the same second independent space 1-22. However, when the first bearing piece 5-20 is located in the first fetching position, the first bearing piece 5-20 extends forwards out of the first rack 5-10. At this moment, the first bearing piece 5-20 can enter the first independent space 1-21, thereby realizing cross-space transportation of the sample or reagent. Since the first switching piece 11-81 is arranged between the adjacent first independent space 1-21 and second independent space 1-22, when the first bearing piece 5-20 moves from the first loading position to the first fetching position, the first bearing piece 5-20 pushes the first switching piece 11-81. In the present embodiment, the first pushing structure 5-30 is arranged at the front end portion of the first bearing piece 5-20, and the first pushing structure 5-30 includes a first rotating piece 5-31 protruding forwards from the first bearing piece 5-20. When the first bearing piece 5-20 moves from the first loading position to the first fetching position, the first rotating piece 5-31 may push the first switching piece 11-81 prior to the first bearing piece 5-20. In the process that the first switching piece 11-81 is pushed away, the first rotating piece 5-31 may convert sliding friction into rolling friction, thereby effectively reducing the friction between the first conveying mechanism 5-01 and the first switching piece 11-81 during rubbing, effectively reducing, on the one hand, the noise of the first switching piece 11-81 in the process of being in the first opening state, and on the other hand, ensuring the service life of the first conveying mechanism 5-01 and the first switching piece 11-81, so that the first conveying mechanism 5-01 can smoothly enter the other from one of the first independent space 1-21 and the second independent space 1-22.

As shown in Figs. 8 to 11, the first division plate 1-11 includes a first plate body 1-111 and a second plate body 1-112 which are arranged at intervals in the first direction. A part of the inner wall of the housing 1-10 and the first plate body 1-111 enclose the first independent space 1-21, and another part of the inner wall of the housing 1-10, the second plate body 1-112 and the second division plate 1-12 enclose the second independent space 1-22. The arrangement of the first plate body 1-111 and the second plate body 1-112 can form physical isolation between adjacent first independent space 1-21 and second independent space 1-22. The first plate body 1-111 is provided with a first conveying window 1-131, the second plate body 1-112 is provided with a second conveying window 1-132 corresponding to the first conveying window 1-131, and the first conveying window 1-131 and the second conveying window 1-132 jointly define the first conveying channel. The first switching piece 11-81 is pivotally arranged on the first plate body 1-111 by means of a first rotating shaft, a first reset member 11-83 is arranged between the first rotating shaft and the corresponding first plate body 1-111 , and the first reset member 11-83 keeps the first switching piece 11-81 in the first closing state under circumstance of applying a first reset force to the first switching piece 11-81. Thus, only when the first pushing structure 5-30 pushes the first switching piece 11-81, the first switching piece 11-81 can switch to the first opening state of opening the first conveying window 1-131. When the first switching piece 11-81 is in the first closing state for closing the first conveying window 1-131, the periphery of the second conveying window 1-132 can be sealed by means of adhesive sealing cotton. This arrangement facilitates improving the isolation effect of the first plate body 1-111 and the second plate body 1-112 on the first independent space 1-21 and the second independent space 1-22, and can also ensure the smoothness of the movement of the first bearing piece 5-20. Preferably, the above first reset member 11-83 is a first torsion spring.

In some other embodiments, the first switching piece is pivotally arranged on the second plate body by means of the first rotating shaft, and the first reset member is arranged between the first rotating shaft and the corresponding second plate body.

In the present embodiment, the first rotating shaft is located below the first bearing piece 5-20. In the above structure, the closer the first bearing piece 5-20 is to the first fetching position, the more the first bearing piece 5-20 stretches to the first rack 5-10, and the worse the stability is. However, since the first rotating shaft is located below the first bearing piece 5-20, after the first conveying mechanism 5-01 pushes away the first switching piece 11-81, the first switching piece 11-81 has a tendency to move toward the first closing state under the action of the first reset force of the first reset member 11-83, so that the first switching piece 11-81 can have an upward supporting force for the first bearing piece 5-20. Therefore, the first bearing piece 5-20 can stably move from the first loading position to the first fetching position, and the stability in the moving process of the first bearing piece 5-20 is increased.

As shown in Figs. 7, 8 and 12, the second conveying mechanism 6-01 includes a second rack 6-31 arranged in the third independent space 1-23, a second bearing piece 6-32 movably arranged on the second rack 6-31 in the first direction, a second pushing structure 6-322 arranged at the front end portion of the second bearing piece 6-32 and an anti-abrasion piece arranged on the second switching piece 11-21. The second bearing piece 6-32 is provided with a second loading position located in the third independent space 1-23, and the second bearing piece 6-32 is provided with a second fetching position in the second independent space 1-22, when the second switching piece 11-21 is pushed to the second opening state by means of the second pushing structure 6-322, the second bearing piece 6-32 extends into the second independent space 1-22 and reaches the second fetching position. The second container 11-30 may be placed on the second bearing piece 6-32, and is transported across the third independent space 1-23 and the second independent space 1-22 through the movement of the second bearing piece 6-32. Moreover, when the second bearing piece 6-32 pushes the second switching piece 11-21, the second bearing piece 6-32 can be in contact fit with the anti-abrasion piece, thereby improving the smoothness of the second bearing piece 6-32 pushing the second switching piece 11-21, so that the second container 11-30 can achieve stable cross-space transportation.

It is to be noted that the front end portion of the second bearing piece 6-32 refers to an end portion, facing the second independent space, of the second bearing piece 6-32 in the reverse direction of the X axis.

As shown in Figs. 7, 8 and 12, the second pushing structure 6-322 includes a second rotating piece 6-3221 protruding forwards from the second bearing piece 6-32. A rotating axis of the second rotating piece 6-3221 extends in the second direction, and the second direction is perpendicular to the first direction on the horizontal plane. Thus, when the second bearing piece 6-32 moves from the second loading position to the second fetching position, the second rotating piece 6-3221 can push the second switching piece 11-21 prior to the second bearing piece 6-32. In the process that the second switching piece 11-21 is pushed away, the second rotating piece 6-3221 may convert the sliding friction into the rolling friction, thereby effectively reducing the friction between the second bearing piece 6-32 and the second switching piece 11-21 during rubbing, effectively reducing, on the one hand, the noise during door opening, and on the other hand, also ensuring the service life of the second bearing piece 6-32 and the second switching piece 11-21, so that the second bearing piece 6-32 can smoothly enter the second independent space 1-22 from the third independent space 1-23.

As shown in Figs. 7, 8 and 12, the anti-abrasion piece includes a third rotating piece 6-40 arranged on the second switching piece 11-21. A rotating axis of the third rotating piece 6-40 is arranged to be parallel to the rotating axis of the second rotating piece 6-3221. When the second bearing piece 6-32 moves from the second loading position to the second fetching position, the second rotating piece 6-3221 can push the second switching piece 11-21, and the third rotating piece 6-40 can be in contact fit with the second bearing piece 6-32. Thus, in the process that the second bearing piece 6-32 pushes the second switching piece 11-21, the third rotating piece 6-40 is first in contact with the end portion of the second bearing piece 6-32, and as the second bearing piece 6-32 continues to move, the opening angle of the second switching piece 11-21 gradually increases, and the third rotating piece 6-40 transitions from the contact fit with the end portion of the second bearing piece 6-32 to the contact fit with a bottom portion of the second bearing piece 6-32. When the third rotating piece 6-40 is in contact fit with the bottom portion of the second bearing piece 6-32, the second switching piece 11-21 is always in the second opening state. By using the above structure, even if the second switching piece 11-21 is set to be small, the second bearing piece 6-32 can also be continuously in contact fit with the third rotating piece 6-40 on the second switching piece 11-21 during the moving process, and the sliding friction is converted into the rolling friction, thereby improving the smoothness of the movement of the second bearing piece 6-32.

As shown in Figs. 7, 8 and 12, the second division plate 1-12 includes a third plate body 1-121 and a fourth plate body 1-122 which are arranged at intervals in the first direction. Another part of the inner wall of the housing 1-10, the first division plate 1-11 and the third plate body 1-121 enclose the second independent space 1-22, and the rest part of the inner wall of the housing 1-10 and the fourth plate body 1-122 enclose the third independent space 1-23. The arrangement of the third plate body 1-121 and the fourth plate body 1-122 can form physical isolation between adjacent second independent space 1-22 and third independent space 1-23. The third plate body 1-121 is provided with a third conveying window 1-133, the fourth plate body 1-122 is provided with a fourth conveying window 1-134 corresponding to the third conveying window 1-133, and the third conveying window 1-133 and the fourth conveying window 1-134 jointly define the second conveying channel. The second switching piece 11-21 is pivotally arranged on the third plate body 1-121 by means of a second rotating shaft, a second reset member 11-22 is arranged between the second rotating shaft and the corresponding third plate body 1-121, and the second reset member 11-22 keeps the second switching piece 11-21 in the second closing state under circumstance of applying a second reset force to the second switching piece 11-21. Thus, the second switching piece 11-21 can be in the second closing state of closing the third conveying window 1-133 when not subject to an external force, and the periphery of the fourth conveying window 1-13 can be sealed by means of the adhesive sealing cotton. In this way, the second independent space 1-22 and the third independent space 1-23 are mutually independently arranged by means of the second switching piece 11-21, so that the risk of cross pollution among the sample, the reagent and a consumable is reduced. Preferably, the above second reset member 11-22 is a second torsion spring.

Specifically, the first rotating piece 5-31, the second rotating piece 6-3221, and the third rotating piece 6-40 of the present embodiment are all preferably roller structures.

In some other embodiments, the second switching piece is pivotally arranged on the fourth plate body by means of the second rotating shaft, and the second reset member is arranged between the second rotating shaft and the corresponding fourth plate body.

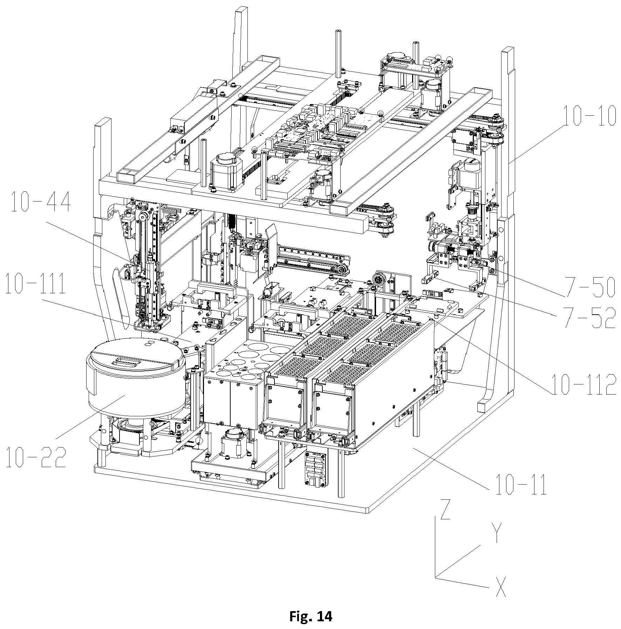

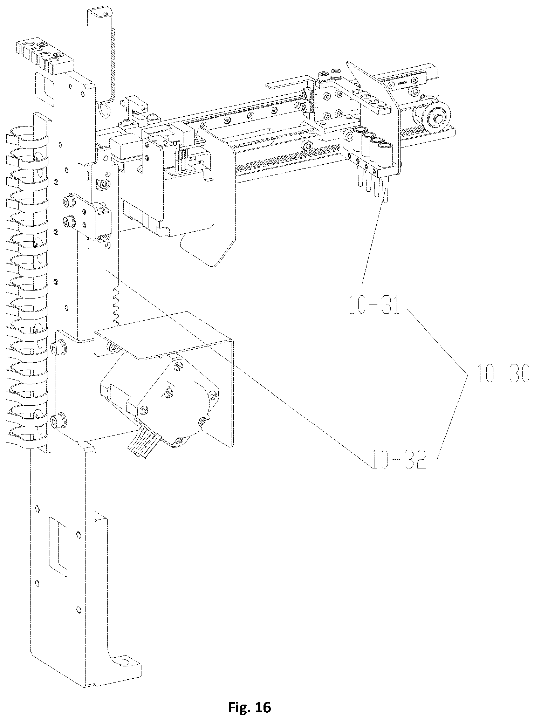

As shown in Figs. 8 to 10 and Figs. 13 to 16, the first container 11-20 is a first well plate. The first well plate is provided with a plurality of first placement holes 11-211, and the plurality of first placement holes 11-211 facilitate placement of different extraction reagents. The reagent preparation apparatus 1.1 includes a first rack body 10-10 and an extraction agent assembly arranged in the first independent space 1-21. The extraction reagent assembly includes a separation reagent assembly 10-30 and an enrichment reagent assembly 10-40. The first rack body 10-10 is provided with a reagent preparation platform 10-11, and the reagent preparation platform 10-11 is provided with a first container placement seat for placing the first container 11-20. The separation reagent assembly 10-30 includes a plurality of liquid injection needles 10-31 movably arranged in the first rack body 10-10 and located above the reagent preparation platform 10-11. Each of the liquid injection needles can be communicated with a corresponding separation reagent, and the plurality of liquid injection needles 10-31 have a first liquid injection position where the plurality of liquid injection needles 10-31 move above the first container placement seat and an avoidance position away from the first container 11-20. The enrichment reagent assembly 10-40 includes a pipetting needle 10-41 and an enrichment reagent bin 10-42 for bearing an elution buffer and a magnetic bead. The pipetting needle 10-41 is movably arranged on the first rack body 10-10 and located above the reagent preparation platform 10-11. The enrichment reagent bin 10-42 is arranged on the reagent preparation platform 10-11, and the pipetting needle 10-41 has a first suction position where the pipetting needle 10-41 move above the enrichment reagent bin 10-42 to load the elution buffer and the magnetic bead and a second liquid injection position where the pipetting needle 10-41 move above the first container placement seat. When the reagent preparation apparatus 1.1 is used for reagent preparation, the plurality of liquid injection needles 10-31 arranged in the first rack body 10-10 are moved to the first liquid injection position above the first container placement seat, causing a separation reagent to be perfused into the corresponding first placement hole 11-211 of the first container 11-20 by means of the liquid injection needle 10-31. The pipetting needle 10-41 arranged in the first rack body 10-10 is moved to the first suction position above the enrichment reagent bin 10-42 to respectively suck the elution buffer and the magnetic bead in the enrichment reagent bin 10-42, and then the pipetting needle 10-41 is moved to the second liquid injection position above the first container placement seat, so as to perfuse the elution buffer and the magnetic bead into the corresponding first placement holes 11-211, that is, the loading process of the extraction reagent can be completed. Compared with the related art, a corresponding quantity of extraction reagents can be prepared according to a required test quantity by use of the reagent preparation apparatus 1.1. Meanwhile, the enrichment reagent bin 10-42 may store different reagent types. That is, different reagents may be loaded according to different sample types, so as to extract multiple sample types.

In the present embodiment, the separation reagent refers to an intermediate reaction reagent, including a lysis buffer, a first washing solution, a second washing solution, and a third washing solution, and used for removing impurities such as cell components other than the analyte in the sample. The enrichment reagent refers to a reagent used for adsorbing and dissolving a purified analyte, and including the magnetic bead and the elution buffer. The plurality of liquid injection needles 10-31 are in one-to-one correspondence with the plurality of first placement holes 11-211. For example, four liquid injection needles 10-31 are arranged in the first rack body 10-10, and can perfuse the lysis buffer, the first washing solution, the second washing solution, and the third washing solution into the four corresponding first placement holes 11-211 of the first container 11-20, respectively.

It is to be noted that six first placement holes 11-211 respectively correspond to the lysis buffer, the first washing solution, the second washing solution, the third washing solution, the magnetic bead, and the elution buffer.

In the present embodiment, when the plurality of liquid injection needles 10-31 are located in the avoidance position, perfusion of the enrichment reagent can be performed by means of the pipetting needle 10-41. In some other specific implementations, the avoidance position of the liquid injection needle 10-31 may be that the liquid injection needle 10-31 is away from the first container 11-20 in the horizontal direction, thereby avoiding interference between the liquid injection needle 10-31 and the pipetting needle 10-41.

As shown in Figs. 8 to 10 and Figs. 13 to 16, the separation reagent assembly 10-30 further includes a perfusion rack 10-32 movably arranged in the first rack body 10-10 in a vertical direction and located above the reagent preparation platform 10-11. The plurality of liquid injection needles 10-31 are movably arranged on the perfusion rack 10-32 in the first direction, the first container placement seat is movably arranged on the reagent preparation platform 10-11 in the second direction, and the second direction is perpendicular to the first direction on the horizontal plane. The first container placement seat has a first working position. When the first container placement seat is in the first working position, the perfusion rack 10-32 can move in the vertical direction so that the plurality of liquid injection needles 10-31 are located in the first liquid injection position. By adopting the perfusion rack 10-32, the plurality of liquid injection needles 10-31 can move in the first direction and in the vertical direction, and then the plurality of liquid injection needles 10-31 can complete a liquid injection operation of perfusing the separation reagent into the first container 11-20.

As shown in Figs. 8 to 10 and Figs. 13 to 15, the enrichment reagent assembly 10-40 further includes a reagent arm 10-44 horizontally and movably arranged on the first rack body 10-10 and located above the reagent preparation platform 10-11. The pipetting needle 10-41 is movably arranged on the reagent arm 10-44 in the vertical direction. The first container placement seat further has a second working position located on one side of the first working position, and when the first container placement seat is located in the second working position, the reagent arm 10-44 can move in the horizontal direction so that the pipetting needle 10-41 is located in the second liquid injection position. Thus, three-axis movement of the pipetting needle 10-41 can be realized, and then the reagent arm 10-44 can be conveniently moved above the enrichment reagent bin 10-42 and above the first container 11-20.

The first container placement seat further has a third working position arranged adjacent to the first working position. A part of the plurality of first placement holes 11-211 are sequentially arranged in two columns of first placement hole groups 10-21 in the first direction, and another part of the plurality of first placement holes 11-211 are spaced apart in the second direction to form a plurality of rows of first placement hole groups 10-21. When the plurality of liquid injection needles 10-31 are located in the first liquid injection position, the first working position corresponds to one column of first placement hole group 10-21, and the third working position corresponds to the other column of first placement hole group 10-21. By using the above arrangement, multiple extraction reagents can be perfused at the same time, so as to enhance the nucleic acid detection capability of the sample analysis device.

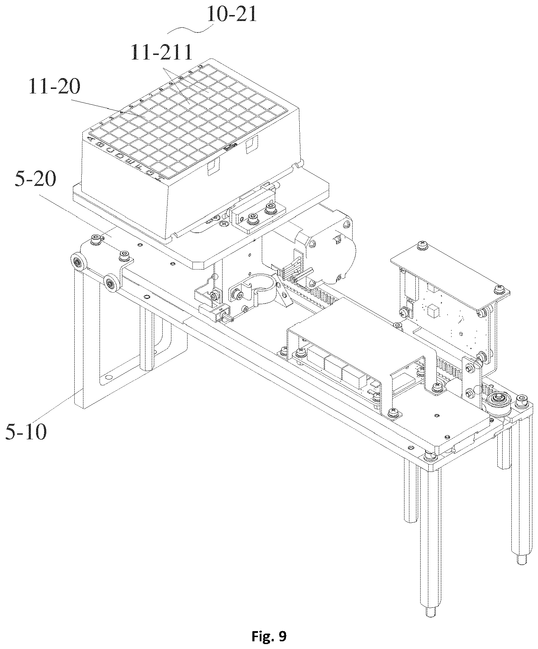

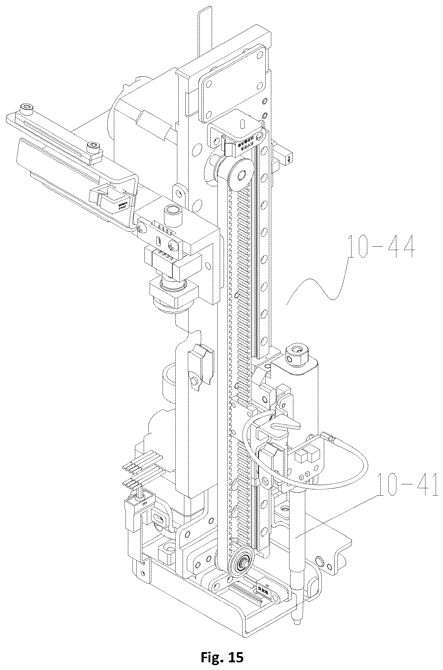

Specifically, as shown in Fig. 9 and Fig. 15, the first container 11-20 has two columns of first placement hole groups 10-21. Each column of the first placement hole group 10-21 consists of six first placement holes 11-211, and the first container 11-20 has eight rows of first placement hole groups 10-21, that is, the first container 11-20 is provided with 8 × 12 first placement holes 11-211 in total. Six first placement holes 11-211 in each column of the first placement hole group 10-21 respectively correspond to the lysis buffer, the first washing solution, the second washing solution, the third washing solution, the magnetic bead, and the elution buffer. Four separation reagents can be perfused into the corresponding four first placement holes 11-211 by use of the four liquid injection needles 10-31, and two enrichment reagents can be perfused into the corresponding two first placement holes 11-211 by use of the pipetting needles 10-41.

In the present embodiment, in order to reduce the volume of the reagent preparation mechanism, the first container 11-20 is configured to be movable in the second direction to fit the liquid injection operation of the liquid injection needle 10-31.

It is to be noted that since the liquid injection needle 10-31 can only perform two-axis movement in the vertical plane and the pipetting needle 10-41 can perform three-axis movement of the space, in order to avoid the interference in the movement process of the perfusion rack 10-32 and the reagent arm 10-44, the first container 11-20 is required to move in the second direction, so that the first container 11-20 can have a plurality of working positions.

As shown in Figs. 8 to 10 and Figs. 13 and 14, the reagent preparation platform 10-11 is provided with a premix position 10-111 for placing a premix plate and a second container placement position 10-112 for placing the second container 11-30. The reagent preparation apparatus 1.1 further includes a preparation reagent bin 10-22 for placing a first reagent bottle and a second reagent bottle. The preparation reagent bin 10-22 is arranged on the reagent preparation platform 10-11, and the pipetting needle 10-41 further has a second suction position where the pipetting needle 10-41 move above the preparation reagent bin 10-22 to suck the amplification reagent in the preparation reagent bin 10-22, a mixing position where the pipetting needle 10-41 move above the premix plate to fill the amplification reagent to the premix plate and a filling position where the pipetting needle 10-41 move above the second container 11-30 to fill a mixed solution in the premix plate to the second container 11-30.

When the reagent preparation apparatus 1.1 is applied to preparing the amplification reagent, the premix plate is placed in the premix position 10-111 of the reagent preparation platform 10-11. The second container 11-30 is placed in the second container placement position 10-112, the pipetting needle 10-41 arranged above the reagent preparation platform 10-11 is moved to the second suction position located above the preparation reagent bin 10-22 to respectively suck a first reagent in the first reagent bottle and a second reagent in the second reagent bottle. Then, the pipetting needle 10-41 is moved to the mixing position above the premix plate, the first reagent and the second reagent are both filled into the premix plate and mixed into the mixed solution, the mixed solution is sucked by use of the pipetting needle 10-41 and moved to the filling position to fill the mixed solution into the second container 11-30, so that the preparation of the amplification reagent can be completed. Compared with a manner of manually preparing the amplification reagent, the method has the advantage of high efficiency.

In the present embodiment, the premix plate is provided with a plurality of premix grooves penetrating an upper surface of the premix plate.

It is to be noted that two types of amplification reagents are included. A first reagent is provided in the first reagent bottle, a second reagent is provided in the second reagent bottle, and the mixed solution formed by mixing the first reagent and the second reagent can be used for amplification of the sample.

The pipetting needle 10-41 may be provided with a fixed pipetting tip, or may be provided with a replaceable pipetting tip, and may be provided with one pipetting tip or may be provided with a plurality of pipetting tips. When the pipetting needle 10-41 is provided with the fixed pipette tip, the reagent preparation mechanism includes two pipetting needles 10-41, and the pipetting tips of the two pipetting needles 10-41 respectively absorb different amplification reagents. When the pipetting needle 10-41 is provided with the replaceable pipette tip, after the pipetting tip sucks the first reagent, the pipetting tip shall be unloaded, and then a new pipetting tip is loaded to suck the second reagent. By use of the above solution, mutual pollution of the first reagent and the second reagent can be avoided.

In the present embodiment, the pipetting needle 10-41 is provided with the replaceable pipetting tip. After the second reagent is sucked by use of the new pipetting tip, the pipetting tip may not be unloaded, and the pipetting tip may be continuously used to uniformly mix the mixed solution by repeatedly sucking and discharging the mixed solution in the premix groove.

Before the amplification reagent is prepared, the usage amounts of the first reagent and the second reagent shall be determined according to the sample amount, and the amplification reagent is quantitatively prepared according to the detection requirement, so that using right after preparation is realized, the problem of reagent deterioration caused by too long mixing standing time is avoided, and reagent waste is also prevented.

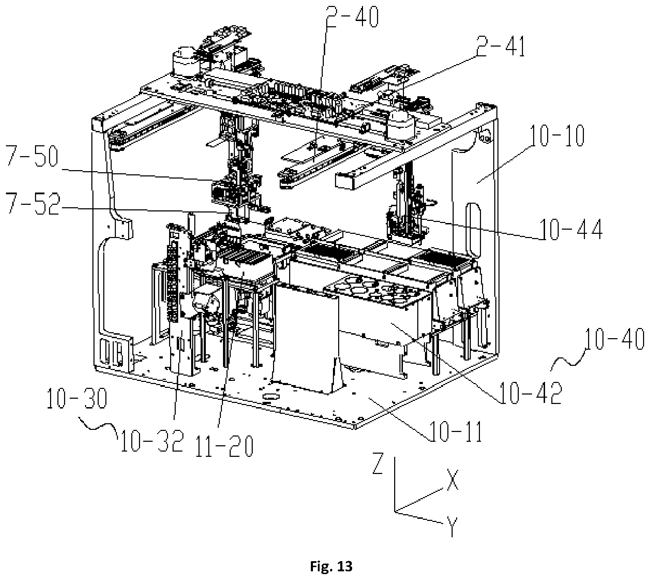

As shown in Figs. 13 to 15, the reagent preparation apparatus 1.1 further includes a transverse guide rail 2-40 and a longitudinal guide rail 2-41. The transverse guide rail 2-40 and the longitudinal guide rail 2-41 are both located above the reagent preparation platform 10-11, and the longitudinal guide rail 2-41 is arranged on the first rack body 10-10 and extends in the first direction. The transverse guide rail 2-40 extends in the second direction perpendicular to the first direction in the horizontal plane, and the transverse guide rail 2-40 is movably arranged below the longitudinal guide rail 2-41 in the extending direction of the longitudinal guide rail 2-41. The reagent arm 10-44 is movably arranged on the transverse guide rail 2-40 in the extending direction of the transverse guide rail 2-40, and the pipetting needle 10-41 is movably arranged on the reagent arm 10-44 in the vertical direction. Thus, the movement of the reagent arm 10-44 in the horizontal direction can be realized by use of the transverse guide rail 2-40 and the longitudinal guide rail 2-41, and then the three-axis movement of the pipetting needle 10-41 can be realized. The advantage of a simple structure is achieved.

Further, the second container 11-30 is a second well plate. The first well plate is provided with a plurality of first placement holes 11-211, the second well plate is provided with a plurality of second placement holes 11-31, and the number of the first placement holes 11-211 is greater than the number of the second placement holes 11-31. Thus, enough extraction reagents can be mixed with the first well plate to meet the usage amount of the extraction reagent. The volume of the plurality of premix grooves is greater than the volume of the plurality of second placement holes 11-31, so that enough amplification reagents can also be mixed.

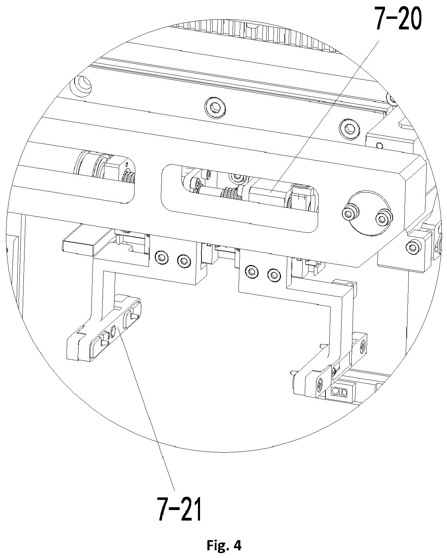

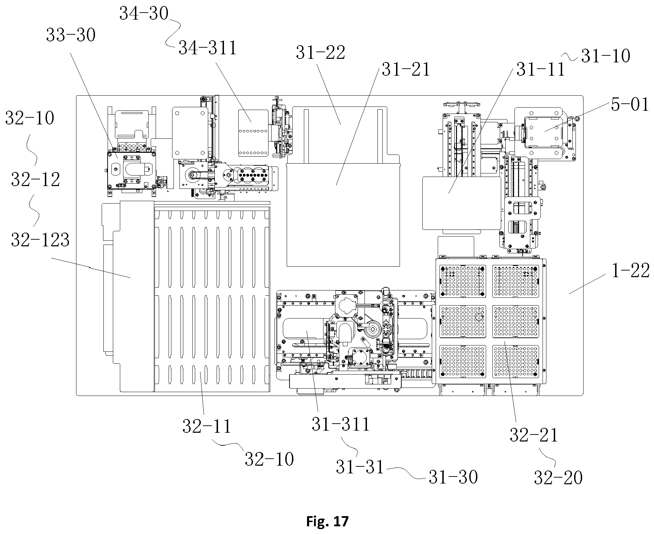

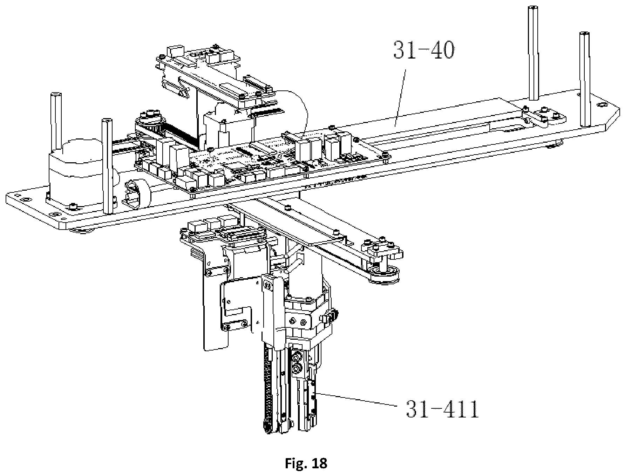

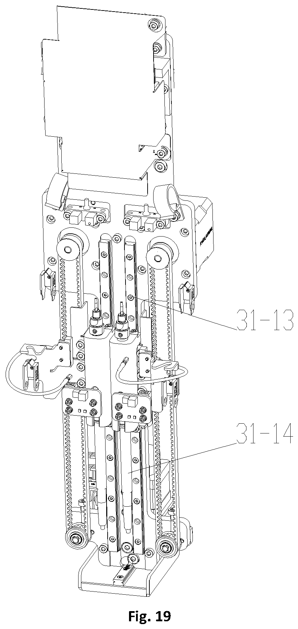

As shown in Fig. 4 and Figs. 17 to 19, the sample processing apparatus 1.2 includes: a sample transfer mechanism 31-10, a filling mechanism 32-20, and an extraction mechanism. The sample transfer mechanism 31-10 includes a transfer rack 31-11 movable in the second direction. The transfer rack 31-11 has a first placement position, and the transfer rack 31-11 has a transfer position and a sample loading position in the moving direction of the transfer rack 31-11. The filling mechanism 32-20 includes a sample arm 31-13. When the transfer rack 31-11 moves to the sample loading position, the sample arm 31-13 moves to the sample loading position and fills the sample or sucks the analyte into the first container 11-20 in the first placement position. The extraction mechanism includes an analyte extraction piece 31-21 and an extraction rack 31-22. The extraction rack 31-22 is movably arranged in the second direction and has a second placement position, and the extraction rack 31-22 has a placement position and an extraction position in the moving direction of the extraction rack 31-22. When the extraction rack 31-22 moves to the placement position and the transfer rack 31-11 moves to the transfer position, the first container 11-20 is moved from the first placement position to the second placement position. When the extraction rack 31-22 moves to the extraction position, the analyte extraction piece 31-21 can extract the analyte from the first container 11-20 in the second placement position. The above analyte is a nucleic acid.

As shown in Fig. 4 and Figs. 17 to 19, the second gripper structure 7-21 places the first container 11-20 filled with the extraction reagent in the first placement position of the transfer position, the sample arm 31-13 fills the sample into the first container 11-20 located in the sample loading position and filled with the extraction reagent, the second gripper structure 7-21 moves the first container 11-20 located in the transfer position to the second placement position located in the placement position of the extraction rack 31-22, the analyte extraction piece 31-21 performs extraction of the nucleic acid from the first container 11-20 in the extraction position, the second gripper structure 7-21 moves the first container 11-20 in the placement position of the extraction rack 31-22 to the first placement position, and the sample arm 31-13 transfers the nucleic acid in the first container 11-20 to the second container 11-30 for amplification detection. During the transportation of the first container 11-20, the second gripper structure 7-21 transfers the first container 11-20 between the sample transfer mechanism 31-10 and the extraction mechanism in the second direction, and fits the movement of the transfer rack 31-11 and the extraction rack 31 - 22 in the first direction perpendicular to the second direction to realize multi-station parallel transmission of the first container 11-20.

In the present embodiment, the sample processing apparatus 1.2 has a buffer position, and the buffer position is arranged on a moving path of the second gripper structure 7-21. When the first container 11-20 of the previous batch completes the extraction of the nucleic acid in the extraction position, and the first container 11-20 of the current batch completes sample loading in the sample loading position, the buffer position is arranged on the moving path of the second gripper structure 7-21, and the first container 11-20 of the current batch that completes sample loading or the first container 11-20 of the previous batch that completes the extraction of the nucleic acid is loaded by use of the buffer position, thus avoiding a transfer conflict of the first container 11-20 between the extraction rack 31-22 and the transfer rack 31-11, improving the transfer efficiency of the first container 11-20 and improving the extraction efficiency of the nucleic acid of the sample processing apparatus 1.2.

When the first container 11-20 of the previous batch completes the extraction of the nucleic acid in the extraction position, and the first container 11-20 of the current batch completes sample loading in the sample loading position, the first container 11-20 that completes the extraction of the nucleic acid can be transferred to the buffer position by use of the second gripper structure 7-21, or the first container 11-20 that completes sample loading can be transferred to the buffer position by use of the second gripper structure 7-21 to avoid the transfer conflict of the first container 11-20 between the extraction rack 31-22 and the transfer rack 31-11.

As shown in Fig. 4 and Figs. 17 to 19, the sample processing apparatus 1.2 further includes: a waste liquid recovery mechanism 34-30. The waste liquid recovery mechanism 34-30 includes a recovery rack, and the recovery rack is provided with a recovery position 34-311. After the sample arm 31-13 transfers the nucleic acid in the first container 11-20 to the second container 11-30, the transfer rack 31-11 moves to the transfer position, the first container 11-20 on the transfer rack 31-11 located in the transfer position is transferred to the recovery position 34-311 on the recovery rack by use of the second gripper structure 7-21, and a waste liquid is pumped from the first container 11-20 and the first container 11-20 are recovered. At least a part of the above recovery position 34-311 forms the buffer position.

As shown in Fig. 4 and Figs. 17 to 19, the sample processing apparatus 1.2 further includes: a storage mechanism 32-10, a transmission mechanism 31-30 and a transport mechanism 31-40. The storage mechanism 32-10 includes a sample storage bin 32-11 for placing a sample tube and a quality control substance storage bin 32-12 for placing a quality control tube that contains a quality control substance. The quality control substance is also called the quality control product, which is a substance such as a quality control product or a standard substance used for monitoring or controlling the quality. The filling mechanism 32-20 further includes a consumable loading mechanism 32-21 for storing a consumable. The sample arm 31-13 can fill the sample in the sample tube or the quality control substance in the quality control tube into the first container 11-20. The sample arm 31-13 is provided with a sample loading needle 31-14, and the sample loading needle 31-14 can be connected with the consumable in a fitting manner. The transmission mechanism 31-30 includes a moving rack 31-31 which is movably arranged. The moving rack 31 - 31 is provided with a bearing position 31-311 for placing the sample tube or the quality control tube, the moving rack 31-31 has a storage transmission position arranged corresponding to the storage mechanism 32-10 and a filling transmission position arranged corresponding to the filling mechanism 32-20, and the moving rack 31-31 can move between the storage transmission position and the filling transmission position. The transport mechanism 31-40 includes a grabbing piece 31-411 capable of transporting the sample tube between the moving rack 31-31 and the sample storage bin 32-11 and transporting the quality control tube between the moving rack 31-31 and the quality control substance storage bin 32-12. The grabbing piece 31-411 can grab the sample tube from the sample storage bin 32-11 or grabbing the quality control tube from the quality control substance storage bin 32-12.

The collected sample is stored by use of the sample tube in the sample storage bin 32-11, and the quality control substance with known information is stored by use of the quality control tube in the quality control substance storage bin 32-12. In the process of nucleic acid detection, when the moving rack 31-31 is located in the storage transmission position, the sample tube is transported from the sample storage bin 32-11 to the bearing position 31-311 of the moving rack 31-31 by use of the grabbing piece 31-411. The moving rack 31-31 moves to the filling transmission position, the sample in the sample tube is filled into the first container 11-20 by using the sample loading needle 31-14. In the detection process of nucleic acid detection, at least once, when the moving rack 31-31 is located in the storage transmission position, the quality control tube is transported from the quality control substance storage bin 32-12 to the bearing position 31-311 of the moving rack 31-31 by use of the grabbing piece 31-411, the moving rack 31-31 moves to the filling transmission position, and the quality control substance in the quality control tube is filled into the first container 11 by use of the sample loading needle 31-14. Then the nucleic acid detection is continuously performed on the collected sample, thus learning whether the sample analysis device works normally according to whether a detection result of the quality control substance matches the known information of the quality control substance, so as to ensure the detection accuracy of the sample analysis device. Moreover, the quality control substance is stored in the quality control tube of the quality control substance storage bin 32-12, causing the extraction of the quality control substance and the collection of the sample to share a set of sample arm 31-13, transmission mechanism 31-30 and transport mechanisms 31-40, which improves the automation degree of loading the quality control substance, simplifies the operation procedures of detection of the quality control substance and shortens the time consumption of the detection of the quality control substance.

In the present embodiment, the sample tube is grabbed from the sample storage bin 32-11 by use of the grabbing piece 31-411, and the quality control tube is grabbed from the quality control substance storage bin 32-12 by use of the grabbing piece 31-411. Thus, in the process of transporting the sample tube from the sample storage bin 32-11 to the bearing position 31-311 of the moving rack 31-31 by use of the grabbing piece 31-411, and in the process of transporting the quality control tube from the quality control substance storage bin 32-12 to the bearing position 31-311 of the moving rack 31-31 by use of the grabbing piece 31-411, the sample tube or the quality control tube is grabbed by means of the grabbing piece 31-411, so that the stability of the sample tube and the quality control tube in the transport process is improved, the sample tube and the quality control tube are prevented from falling during the transport process, and the normal operation of a loading apparatus of an object to be detected is ensured.

Moreover, due to the long operation time of manually loading the quality control substance, and considering the working time and rest time of an inspector, the number of times of manually loading the quality control substance is limited, and the loading frequency cannot meet the requirement of detecting the quality control substance in every batch of nucleic acid detection.

In the present embodiment, the first container 11-20 is filled with the quality control substance for each batch of nucleic acid detection, so that during each batch of nucleic acid detection, it can be learned whether the sample analysis device works normally in this batch of nucleic acid detection according to whether the detection result of the quality control substance in this batch matches the known information of the quality control substance, so as to learn whether the sample analysis device works normally in each batch of nucleic acid detection, thereby ensuring the detection accuracy of each batch of nucleic acid detection and improving the detection precision.

In the present embodiment, the quality control tube includes a first quality control tube body and a second quality control tube body, and the first quality control tube body and the second quality control tube body are respectively used for storing a positive quality control substance and a negative quality control substance. The quality control tube includes the first quality control tube body for storing the positive quality control substance and the second quality control tube body for storing the negative quality control substance. Therefore, in each batch of nucleic acid detection, the positive quality control substance and the negative quality control substance can be filled into the first container 11-20, accordingly the detection result of the positive quality control substance and the detection result of the negative quality control substance in each batch of nucleic acid detection by the sample analysis device can be obtained, and whether the positive quality control substance and the negative quality control substance are detected correctly by the sample analysis device can be used to ensure the detection accuracy of each batch of nucleic acid detection.

The quality control tube also includes a third quality control tube body, and the third quality control tube body is used for storing an exogenous internal standard.

It is to be noted that the first quality control tube body includes a strong-positive quality control tube body and a weak-positive quality control tube body. The strong-positive quality control tube body and the weak-positive quality control tube body are used for storing a strong-positive quality control substance and a weak-positive quality control substance, respectively; therefore, in each batch of nucleic acid detection, the strong-positive quality control substance, the weak-positive quality control substance and the negative quality control substance can be filled into the first container 11-20, and whether the strong-positive quality control substance, the weak-positive quality control substance and the negative quality control substance are detected correctly by the sample analysis device can be used to ensure the detection accuracy of each batch of nucleic acid detection.

In the present embodiment, the storage mechanism 32-10 further includes a refrigerating unit for refrigerating the quality control tube. The quality control tube is refrigerated by use of the refrigerating unit, thus avoiding the quality control substance in the quality control tube from failing, maintaining the stability of the quality control substance, ensuring that the detection result of the quality control substance by the sample analysis device can reflect whether the sample analysis device works normally, ensuring the detection accuracy of nucleic acid detection, and improving the detection precision.

As shown in Fig. 4 and Figs. 17 to 19, the quality control substance storage bin 32-12 is provided with a bin door 32-123 which can be opened and closed. When the grabbing piece 31-411 needs to grab the quality control tube, the bin door 32-123 is opened and the grabbing piece 31-411 grabs the quality control tube from an inner cavity of the quality control substance storage bin 32-12. When the grabbing piece 31-411 does not need to grab the quality control tube, the bin door 32-123 is closed, so that the bin door 32-123 seals the inner cavity of the quality control substance storage bin 32-12, and the refrigeration effect of the quality control substance storage bin 32-12 is ensured. The sample storage bin 32-11 and the quality control substance storage bin 32-12 are arranged independently. Since the sample storage bin 32-11 is an open structure, the sample storage bin 32-11 and the quality control substance storage bin 32-12 are independently arranged, and the sample storage bin 32-11 and the quality control substance storage bin 32-12 are controlled respectively, when the door 32-123 is closed, the pollution between the sample tube in the sample storage bin 32-11 and the quality control tube in the quality control substance storage bin 32-12 can be avoided, the detection accuracy of nucleic acid detection can be ensured, and the detection precision can be improved.

It is to be noted that the sample storage bin 32-11 and the quality control substance storage bin 32-12 are independently arranged, which means that a storage condition of the sample storage bin 32-11 is independent from a storage condition of the quality control substance storage bin 32-12, the sample storage bin 32-11 and the quality control substance storage bin 32-12 occupy a certain independent space, and the two are not mixed, which facilitates respective management of the sample tube and the quality control tube.

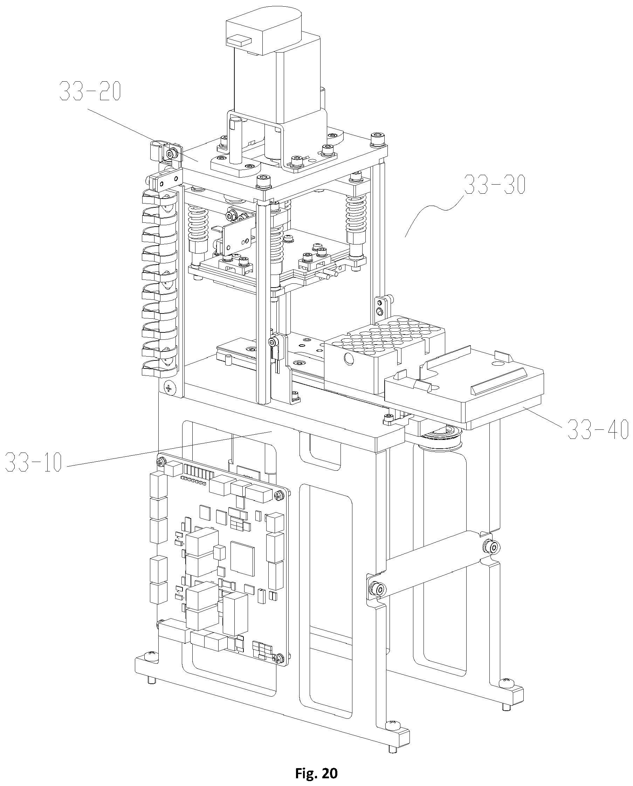

As shown in Figs. 4, 7 and 20, the sample analysis device further includes a sample processing gripper 7-20. The sample processing gripper 7-20 is provided with a second gripper structure 7-21. The sample processing apparatus 1.2 further includes a film sealing mechanism 33-30 arranged in the second independent space 1-22. Herein, the film sealing mechanism 33-30 includes: a third rack 33-10, a hot-pressing mechanism 33-20 and a bearing mechanism 33-40. The hot-pressing mechanism 33-20 is movably arranged on the third rack 33-10 in the vertical direction, and the second gripper structure 7-21 is arranged on one side of the hot-pressing mechanism 33-20. The bearing mechanism 33-40 includes a first placement area and a second placement area which are adjacently arranged. The bearing mechanism 33-40 is movably arranged between the hot-pressing mechanism 33-20 and the second gripper structure 7-21, causing the bearing mechanism 33-40 having a hot-pressing position where the first placement area is in fitting cooperation with the hot-pressing mechanism 33-20, a clamping position where the second placement area is in fitting cooperation with the second gripper structure 7-21, and an assembly position where the first placement area is in fitting cooperation with the second gripper structure. The first placement area is configured to place the second container 11-30, the second container 11-30 is configured to place the mixture to be detected, and the second placement area is configured to place a cover body 11-40.

The above cover body 11-40 includes a frame and a sealing film arranged on the frame. The hot-pressing mechanism 33-20 can seal the sealing film on the second container 11-30 by hot pressing, so that the sealing film can seal each second placement hole 11-31. When film sealing, the bearing mechanism 33-40 is moved to the clamping position, and at this moment, the second gripper structure 7-21 can pick up the cover body 11-40 from the second placement area. Then the bearing mechanism 33-40 is moved to the assembly position, and at this moment, the second gripper structure 7-21 can place the cover body 11-40 on the second container 11-30 where the mixture to be detected is placed. Finally, the bearing mechanism 33 is moved to the hot-pressing position, and at this moment, the hot-pressing mechanism 33-20 can complete the film sealing operation. The above structure can realize the assembly and film sealing operation of the cover body 11-40 and the second container 11-30 by means of the film sealing mechanism 33-30, causing the film sealing mechanism 33-30 to be more functional. Meanwhile, the above structure can realize the film sealing operation of the cover body 11-40 and the second container 11-30 only by arranging one film sealing mechanism 33-30. The steps are simple, the film sealing efficiency is high, and no other auxiliary equipment is required.

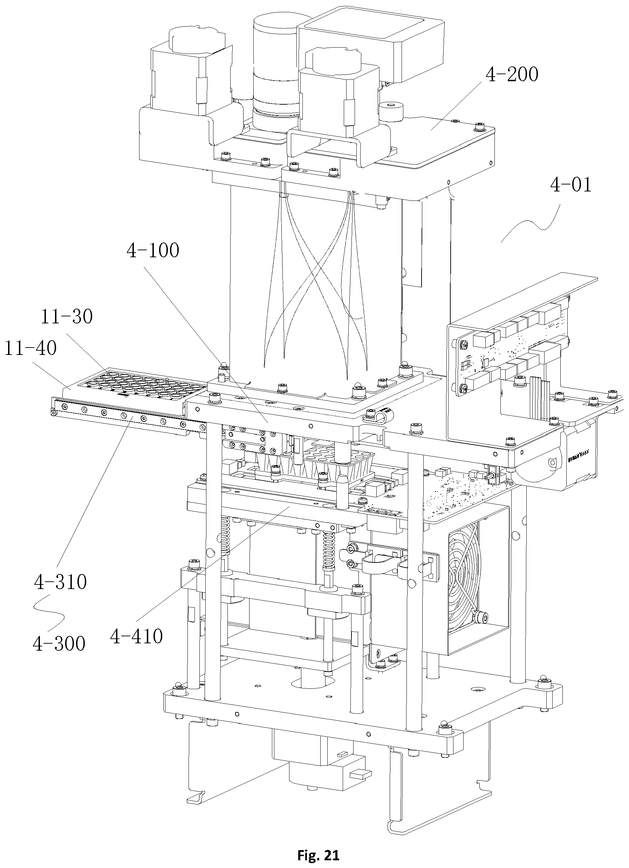

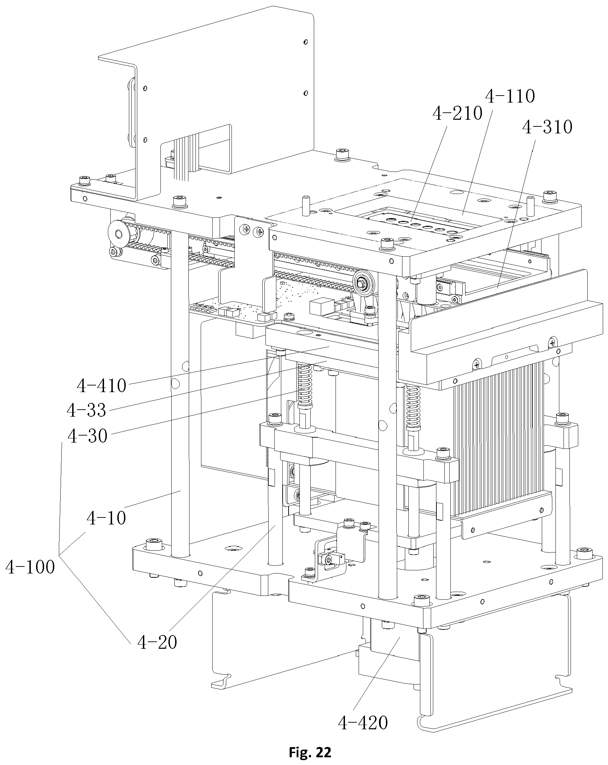

As shown in Figs. 8, 21 and 22, the analysis apparatus 1.3 includes a plurality of amplification mechanisms 4-01 sequentially arranged in the second direction, Each amplification mechanism 4-01 includes: a second rack body 4-100, a detection apparatus 4-200, a reagent loading apparatus 4-300 and a temperature control assembly 4-410. The second rack body 4-100 is provided with a detection window 4-110. The detection apparatus 4-200 is arranged on the second rack body 4-100 and located at the detection window 4-110, so as to detect the reagent by means of the detection window 4-110. The reagent loading apparatus 4-300 includes a bracket 4-310. The second container 11-30 is detachably arranged on the bracket 4-310. The bracket 4-310 is movably arranged on the second rack body 4-100 in the first direction, and the second direction is perpendicular to the first direction in the horizontal plane. The second container 11-30 moves along with the bracket 4-310 and has a storage position located below the detection window 4-110 and a loading position where the second container 11-30 moves out of the second rack body 4-100. The temperature control assembly 4-410 is movably arranged on the second rack body 4-100 in the vertical direction and can drive the second container 11-30 to ascend and descend, causing the second container 11-30 to be lifted to a detection position in butt joint fit with the detection apparatus 4-200. The second container 11-30 can also contain the mixture to be detected. The second container 11-30 moves along with the bracket 4-310, causing the second container 11-30 having the storage position located below the detection window 4-110 and the loading position where the second container 11-30 moves out of the second rack body 4-100. The second container 11-30 containing the mixture to be detected is transported from the outside of the analysis apparatus 1.3 to the bracket 4-310, and the second container 11-30 containing the mixture to be detected moves between the storage position and the loading position along with the bracket 4-310. The temperature control assembly 4-410 is movably arranged on the second rack body 4-100 in the vertical direction and can drive the second container 11-30 to ascend and descend, causing the second container 11-30 to be lifted to the detection position in butt joint fit with the detection apparatus 4-200. Thus, the second container 11-30 located in the detection position can reduce a distance between the temperature control assembly 4-410 and the second container 11-30 containing the mixture to be detected, so that the mixture to be detected in the second container 11-30 can be better heated to achieve the purpose of amplification, and the second container 11-30 is located in the detection position at this moment, so the detection apparatus 4-200 can detect the mixture to be detected in the second container 11-30 to judge the amplification effect of the mixture to be detected. Thus, the detection apparatus 4-200, the reagent loading apparatus 4-300 and the temperature control assembly 4-410 are vertically arranged by means of the second rack body 4-100, thus reducing the floor space of the analysis apparatus 1.3, causing the structure to be compact, and improving the space utilization rate, and a plurality of amplification mechanisms 4-01 can be arranged side by side and processed in parallel to achieve the goal of high throughput. It is to be noted that the above butt joint fit can be a contact fit and can also be a fit with a certain gap.

As shown in Figs. 8, 21 and 22, the second rack body 4-100 includes a frame assembly. The frame assembly includes an outer frame 4-10, an inner frame 4-20 and a lifting frame member 4-30. The outer frame 4-10 includes a top plate and a bottom plate arranged at an interval, and a first support and a second support arranged between the top plate and the bottom plate. The detection window 4-110 is arranged on the top plate of the frame assembly. The bracket 4-310 is arranged on the top plate. A lifting space is enclosed among the top plate, the bottom plate, the first support and the second support. The inner frame 4-20 includes a third support and a fourth support arranged between the bottom plate and the top plate. The lifting frame member 4-30 is located in the lifting space in a lifting manner. The lifting frame member 4-30 includes a lifting plate penetrating the third support and the fourth support in a lifting manner, a first support guide penetrating the third support, a second support guide penetrating the fourth support, and a bearing beam 4-33 connected to the first support guide and the second support guide in a floatable manner. Thus, the first support guide and the second support guide can play a guiding role during the lifting of the lifting frame member 4-30, causing the lifting of the lifting frame member 4-30 to be more stable. The first support guide and the second support guide are both connected with the lifting plate, and a placement space is enclosed among the third support, the fourth support and the lifting plate. A driving assembly 4-420 is arranged on the bottom plate and is in driving connection with the lifting plate. The temperature control assembly 4-410 can be put into the placement space, so that the first support guide and the second support guide can guide and simultaneously support the temperature control assembly 4-410 to be supported by the lifting frame member 4-30. The driving assembly 4-420 drives the lifting plate to ascend and descend, so that the lifting frame member 4-30 drives the temperature control assembly 4-410 to ascend and descend. The second container 11-30 containing the mixture to be detected can be placed on the top plate, the lifting frame member 4-30 drives the temperature control assembly 4-410 to ascend, and the temperature control assembly 4-410 controls the temperature of the second container 11-30 containing the mixture to be detected on the top plate. Thus, the inner frame 4-20 is embedded in the outer frame 4-10, and the lifting frame member 4-30 is embedded in the inner frame 4-20. This mutually embedded structure causes the structure of the frame assembly to be compact and reduces the volume and floor space of the temperature control assembly 4-410. Moreover, since the lifting frame member 4-30 is embedded in the inner frame 4-20 for ascending and descending, the lifting space in the vertical direction is fully utilized, the horizontal movement of the temperature control assembly 4-410 in the related art is avoided, the floor space of the temperature control assembly 4-410 is reduced, and the floor space of the analysis apparatus 1.3 provided with the temperature control assembly 4-410 is reduced, so that the floor space required for arranging a plurality of amplification mechanisms 4-01 is reduced, and the implementation of the high throughput goal is facilitated.

As shown in Figs. 8, 21 and 22, after the bearing beam 4-33 drives the temperature control assembly 4-410 to ascend to a position to abut against the second container 11-30 containing the mixture to be detected, since the bearing beam 4-33 can float, when the temperature control assembly 4-410 abuts against the second container 11-30 containing the mixture to be detected, the second container 11-30 or another apparatus abutting against an upper part of the second container 11-30 can be prevented from being damaged caused by excessive abutting force borne by the second container 11-30, thus achieving the buffer effect.

The temperature control assembly 4-410 further includes a perforated plate seat, a radiator arranged below the perforated plate seat, a circuit board and a uniform temperature plate. The top of the radiator is provided with a flange, and the flange surrounds the circumferential outer side of the top of the radiator, so that the upper surface of the bearing beam 4-33 of the frame assembly can abut against the flange of the radiator to hold the radiator and drive the radiator to ascend and descend. When the second container 11-30 is in the storage position, the second container 11-30, the detection window 4-110, the perforated plate seat, a heat cover 4-210 and the temperature control assembly 4-410 are in the same vertical direction.

As shown in Figs. 8, 21 and 22, in the present embodiment, the detection window 4-110 is a rectangular opening penetrating an upper surface of the top plate and a lower surface of the top plate, and the heat cover 4-210 is arranged on the lower surface of the top plate and covers the detection window 4-110. The detection apparatus 4-200 further includes an optical fiber installation structure arranged on the top plate. The optical fiber installation structure includes an installation seat and a plurality of optical fiber installation posts arranged on the installation seat, and the optical fiber installation post detects the mixture to be detected in the second container 11-30 using an optical signal. The heat cover 4-210 is provided with a plurality of avoidance holes in one-to-one correspondence with the plurality of second placement holes 11-31 in the second container 11-30, so that the optical fiber installation post of the detection apparatus 4-200 can extend into the avoidance hole of the heat cover 4-210 and analyze the mixture to be detected in the second placement hole 11-31 in the second container 11-30. During the analysis, the detection apparatus 4-200 collects a fluorescence signal of the mixture to be detected to form the detection result. After the amplification detection and analysis is completed, the temperature control assembly 4-410 descends, and the second container 11-30 descends for a distance with the temperature control assembly 4-410 to reach the storage position. At this moment, the second container 11-30 is held by the bracket 4-310 and separated from the temperature control assembly 4-410. The bracket 4-310 drives the second container 11-30 to move transversely, causing the second container 11-30 to be in the loading position, and the third gripper structure 7-31 grabs and moves the second container 11-30 to the recovery mechanism to recycle the second container 11-30, thus completing a primary amplification detection and analysis process of the second container 11-30.

It is also to be noted that, in the present invention, the first bearing piece 5-20, the second bearing piece 6-32, the liquid injection needle 10-31, the pipetting needle 10-41, the perfusion rack 10-32, the first container placement seat, the reagent arm 10-44, the transfer rack 31-11 , the sample arm 31-13, the extraction rack 31-22, the moving rack 31-31, the reagent preparation gripper 7-50, the first gripper structure 7-52, the sample processing gripper 7-20, the second gripper structure 7-21, the analysis gripper 7-30, the third gripper structure 7-31, the hot-pressing mechanism 33-20, the bearing mechanism 33-40, the bracket 4-310 and the temperature control assembly 4-410 which are movably arranged can be moved by a motor driving a drive mechanism. In order for moving smoothly according to a predetermined trajectory when moving, a guide rail structure is also arranged.

In the description of the invention, it is to be understood that orientation or position relationships indicated by terms "front", "back", "upper", "lower", "left", "right", "transverse", "longitudinal", "vertical", "horizontal", "top", "bottom" and the like are orientation or position relationships shown in the drawings usually, are adopted not to indicate or imply that indicated devices or components must be in specific orientations or structured and operated in specific orientations but only to conveniently describe the invention and simplify descriptions and thus should not be understood as limits to the scope of protection of invention. The orientations "inner" and "outer" are the inside and the outside relative to an outline of each part.

For convenient description, spatially relativity terms such as "on", "above", "on the surface of", "on the top of" may be used herein to describe the spatial positional relationship of one device or one feature to other devices or features as shown in the drawings. It will be understood that the spatially relativity terms are intended to encompass different orientations used or operated in addition to the orientations of the devices described in the drawings. For example, if the device in the drawings is inverted, the device described as "on other devices or configurations" or "above other devices or configurations" will then be positioned "under other devices or configurations" or "below other devices or configurations." Thus, the exemplary term "above" may include both orientations of "above" and "below". The device may also be positioned in other different ways (rotated 90° or at other orientations) and the spatially relativity description used herein is interpreted accordingly.

In addition, it should be noted that terms "first", "second" and the like are used for limiting the parts, for facilitating distinguishing corresponding parts only. Unless otherwise stated, the above terms do not have special meanings, and accordingly should not be understood to limit the scope of protection of the invention.

The above is only the preferred embodiments of the invention and is not used to limit the invention. For those skilled in the art, there may be various changes and variations in the invention. Any modifications, equivalent replacements, improvements and the like made within the spirit and principle of the present invention shall fall within the scope of protection of the present invention.

## Claims

1. A sample analysis device, comprising:
a housing (1-10), wherein a first independent space (1-21), a second independent space (1-22) and a third independent space (1-23) are sequentially arranged in the housing (1-10) at intervals;
a reagent preparation apparatus (1.1), configured to prepare an extraction reagent and an amplification reagent, the reagent preparation apparatus (1.1) being arranged in the first independent space (1-21);
a sample processing apparatus (1.2), configured to extract an analyte from a sample and combine the analyte with the amplification reagent to form a mixture to be detected, the sample processing apparatus (1.2) being arranged in the second independent space (1-22), a first conveying channel being arranged between the reagent preparation apparatus (1.1) and the sample processing apparatus (1.2), a first switching piece (11-81) being arranged at the first conveying channel, the first switching piece (11-81) having a first opening state and a first closing state, the first switching piece (11-81) opening the first conveying channel when in the first opening state, so that the first independent space (1-21) is communicated with the second independent space (1-22), and the first switching piece (11-81) closing the first conveying channel when in the first closing state, so that the first independent space (1-21) and the second independent space (1-22) are independent from each other; and
an analysis apparatus (1.3), configured to analyze the mixture to be detected, the analysis apparatus (1.3) being arranged in the third independent space (1-23), a second conveying channel being arranged between the analysis apparatus (1.3) and the sample processing apparatus (1.2), a second switching piece (11-21) being arranged at the second conveying channel, the second switching piece (11-21) having a second opening state and a second closing state, the second switching piece (11-21) opening the second conveying channel when in the second opening state, so that the second independent space (1-22) is communicated with the third independent space (1-23), and the second switching piece (11-21) closing the second conveying channel when in the second closing state, so that the second independent space (1-22) and the third independent space (1-23) are independent from each other.

2. The sample analysis device according to claim 1, wherein the reagent preparation apparatus (1.1) further comprises a first container (11-20) and a second container (11-30), the first container (11-20) being configured to place the extraction reagent, the second container (11-30) being configured to place the amplification reagent, a first division plate (1-11) and a second division plate (1-12) being arranged in the housing (1-10) at intervals, the first division plate (1-11) and the second division plate (1-12) separating an inner space of the housing (1-10) to form the first independent space (1-21), the second independent space (1-22) and the third independent space (1-23), the first conveying channel being arranged on the first division plate (1-11), the second conveying channel being arranged on the second division plate (1-12), the first container (11-20) and the second container (11-30) in the first independent space (1-21) being conveyed into the second independent space (1-22) by the first conveying channel, and the second container (11-30) in the second independent space (1-22) being conveyed into the third independent space (1-23) by the second conveying channel.

3. The sample analysis device according to claim 2, wherein the sample analysis device further comprises a first conveying mechanism (5-01) arranged in the first independent space (1-21) or in the second independent space (1-22) and a second conveying mechanism (6-01) arranged in the second independent space (1-22) or in the third independent space (1-23), the first conveying mechanism (5-01) conveying the first container (11-20) and the second container (11-30) through the first conveying channel and driving the first switching piece (11-81) to switch between the first opening state and the first closing state, and the second conveying mechanism (6-01) conveying the second container (11-30) through the second conveying channel and driving the second switching piece (11-21) to switch between the second opening state and the second closing state.

4. The sample analysis device according to claim 3, wherein the first conveying mechanism (5-01) comprises a first rack (5-10) arranged in the second independent space (1 -22), a first bearing piece (5-20) movably arranged on the first rack (5-10) in a first direction and a first pushing structure (5-30) arranged at a front end portion of the first bearing piece (5-20), the first direction being a direction in which the first independent space (1-21), the second independent space (1-22) and the third independent space (1-23) are sequentially arranged, the first bearing piece (5-20) being provided with a first loading position located in the second independent space (1-22), and the first bearing piece (5-20) being provided with a first fetching position in the first independent space (1-21), when the first switching piece (11-81) is pushed to the first opening state by the first pushing structure (5-30), the first bearing piece (5-20) extends into the first independent space (1-21) and reaches the first fetching position.

5. The sample analysis device according to claim 4, wherein the first pushing structure (5-30) comprises a first rotating piece (5-31) protruding forwards from the first bearing piece (5-20), a rotating axis of the first rotating piece (5-31) extending in a second direction, the second direction being perpendicular to the first direction on a horizontal plane, and when the first bearing piece (5-20) moves from the first loading position to the first fetching position, the first rotating piece (5-31) being capable of pushing the first switching piece (11-81).

6. The sample analysis device according to claim 4, wherein
the first division plate (1-11) comprises a first plate body (1-111) and a second plate body (1-112) which are arranged at intervals in the first direction, a part of an inner wall of the housing (1-10) and the first plate body (1-111) enclosing the first independent space (1-21), and another part of the inner wall of the housing (1-10), the second plate body (1-112) and the second division plate (1-12) enclosing the second independent space (1-22); and
the first plate body (1-111) is provided with a first conveying window (1-131), the second plate body (1-112) being provided with a second conveying window (1-132) corresponding to the first conveying window (1-131), the first conveying window (1-131) and the second conveying window (1-132) jointly defining the first conveying channel, the first switching piece (11-81) being pivotally arranged on the first plate body (1-111) or the second plate body (1-112) by a first rotating shaft, a first reset member (11-83) being arranged between the first rotating shaft and the first plate body (1-111) or the first rotating shaft and the second plate body (1-112), and the first reset member (11-83) keeping the first switching piece (11-81) in the first closing state under circumstance of applying a first reset force to the first switching piece (11-81).

7. The sample analysis device according to claim 3, wherein the second conveying mechanism (6-01) comprises a second rack (6-31) arranged in the third independent space (1-23), a second bearing piece (6-32) movably arranged on the second rack (6-31) in a first direction, a second pushing structure (6-322) arranged at a front end portion of the second bearing piece (6-32) and an anti-abrasion piece arranged on the second switching piece (11-21), the second bearing piece (6-32) being provided with a second loading position located in the third independent space (1-23), and the second bearing piece (6-32) being provided with a second fetching position in the second independent space (1-22), when the second switching piece (11-21) is pushed to the second opening state by the second pushing structure (6-322), the second bearing piece (6-32) extends into the second independent space (1-22) and reaches the second fetching position.

8. The sample analysis device according to claim 7, wherein the second pushing structure (6-322) comprises a second rotating piece (6-3221) protruding forwards from the second bearing piece (6-32), a rotating axis of the second rotating piece (6-3221) extending in a second direction, the second direction being perpendicular to the first direction on a horizontal plane, the anti-abrasion piece comprising a third rotating piece (6-40) arranged on the second switching piece (11-21), a rotating axis of the third rotating piece (6-40) being arranged to be parallel to the rotating axis of the second rotating piece (6-3221), when the second bearing piece (6-32) moves from the second loading position to the second fetching position, the second rotating piece (6-3221) being capable of pushing the second switching piece (11-21), and the third rotating piece (6-40) being capable of being in contact fit with the second bearing piece (6-32).

9. The sample analysis device according to claim 3, wherein
the second division plate (1-12) comprises a third plate body (1-121) and a fourth plate body (1-122) which are arranged at intervals in a first direction, another part of the inner wall of the housing (1-10), the first division plate (1-11) and the third plate body (1-121) enclosing the second independent space (1-22), and the rest part of the inner wall of the housing (1-10) and the fourth plate body (1-122) enclosing the third independent space (1-23); and
the third plate body (1-121) is provided with a third conveying window (1-133), the fourth plate body (1-122) being provided with a fourth conveying window (1-134) corresponding to the third conveying window (1-133), the third conveying window (1-133) and the fourth conveying window (1-134) jointly defining the second conveying channel, the second switching piece (11-21) being pivotally arranged on the third plate body (1-121) or the fourth plate body (1-122) by a second rotating shaft, a second reset member (11-22) being arranged between the second rotating shaft and the third plate body (1-121) or the second rotating shaft and the fourth plate body (1-122), and the second reset member (11-22) keeping the second switching piece (11-21) in the second closing state under circumstance of applying a second reset force to the second switching piece (11-21).

10. The sample analysis device according to claim 2, wherein
the first container (11-20) is a first well plate, a plurality of first placement holes (11-211) being formed in the first well plate, the reagent preparation apparatus (1.1) comprising a first rack body (10-10) and an extraction reagent assembly arranged in the first independent space (1-21), and the extraction reagent assembly comprising a separation reagent assembly (10-30) and an enrichment reagent assembly (10-40);
the first rack body (10-10) is provided with a reagent preparation platform (10-11), the reagent preparation platform (10-11) being provided with a first container placement seat for placing the first container (11-20);
the separation reagent assembly (10-30) comprises a plurality of liquid injection needles (10-31) movably arranged in the first rack body (10-10) and located above the reagent preparation platform (10-11), each of the plurality of liquid injection needles (10-31) being capable of being communicated with a corresponding separation reagent, and the plurality of liquid injection needles (10-31) having a first liquid injection position for moving above the first container placement seat and an avoidance position away from the first container (11-20); and
the enrichment reagent assembly (10-40) comprises a pipetting needle (10-41) and an enrichment reagent bin (10-42) for bearing an elution buffer and a magnetic bead, the pipetting needle (10-41) being movably arranged on the first rack body (10-10) and located above the reagent preparation platform (10-11), the enrichment reagent bin (10-42) being arranged on the reagent preparation platform (10-11), and the pipetting needle (10-41) having a first suction position for moving above the enrichment reagent bin (10-42) to load the elution buffer and the magnetic bead and a second liquid injection position for moving above the first container placement seat.

11. The sample analysis device according to claim 10, wherein
the separation reagent assembly (10-30) further comprises a perfusion rack (10-32) movably arranged in the first rack body (10-10) in a vertical direction and located above the reagent preparation platform (10-11), the plurality of liquid injection needles (10-31) being movably arranged on the perfusion rack (10-32) in a first direction, the first container placement seat being movably arranged on the reagent preparation platform (10-11) in a second direction, the second direction being perpendicular to the first direction on the horizontal plane, the first container placement seat having a first working position, and when the first container placement seat is in the first working position, the perfusion rack (10-32) being capable of moving in a vertical direction so that the plurality of liquid injection needles (10-31) are located in the first liquid injection position; and
the enrichment reagent assembly (10-40) further comprises a reagent arm (10-44) horizontally and movably arranged on the first rack body (10-10) and located above the reagent preparation platform (10-11), the pipetting needle (10-41) being movably arranged on the reagent arm (10-44) in the vertical direction, the first container placement seat further having a second working position located on a side of the first working position, and when the first container placement seat is located in the second working position, the reagent arm (10-44) being capable of moving in the horizontal direction so that the pipetting needle (10-41) is located in the second liquid injection position.

12. The sample analysis device according to claim 11, wherein
the reagent preparation platform (10-11) is provided with a premix position (10-111) for placing a premix plate and a second container placement position (10-112) for placing the second container (11-30); and
the reagent preparation apparatus (1.1) further comprises a preparation reagent bin (10-22) for placing a first reagent bottle and a second reagent bottle, the preparation reagent bin (10-22) being arranged on the reagent preparation platform (10-11), and the pipetting needle (10-41) further having a second suction position where the pipetting needle (10-41) move above the preparation reagent bin (10-22) to suck the amplification reagent in the preparation reagent bin (10-22), a mixing position where the pipetting needle (10-41) move above the premix plate to fill the amplification reagent to the premix plate and a filling position where the pipetting needle (10-41) move above the second container (11-30) to fill a mixed solution in the premix plate to the second container (11-30).

13. The sample analysis device according to claim 10, wherein the second container (11-30) is a second well plate, the second well plate being provided with a plurality of second placement holes (11-31), and a number of the first placement holes (11-211) being greater than a number of the second placement holes (11-31).

14. The sample analysis device according to claim 2, wherein the sample processing apparatus (1.2) comprises:
a sample transfer mechanism (31-10), comprising a transfer rack (31-11) movable in a second direction, the transfer rack (31-11) having a first placement position, and the transfer rack (31-11) having a transfer position and a sample loading position in a moving direction of the transfer rack (31-11);
a filling mechanism (32-20), the filling mechanism (32-20) comprising a sample arm (31-13), and when the transfer rack (31-11) moves to the sample loading position, the sample arm (31-13) moves to the sample loading position and fills a sample or sucks the analyte in the first container (11-20) in the first placement position; and
an extraction mechanism, comprising an analyte extraction piece (31-21) and an extraction rack (31-22), the extraction rack (31-22) being movably arranged in the second direction and having a second placement position, the extraction rack (31-22) having a placement position and an extraction position in a moving direction of the extraction rack (31-22), when the extraction rack (31-22) moves to the placement position and the transfer rack (31-11) moves to the transfer position, the first container (11-20) being moved from the first placement position to the second placement position, and when the extraction rack (31-22) moves to the extraction position, the analyte extraction piece (31-21) being capable of extracting the analyte from the first container (11-20) in the second placement position;
and/or
wherein the sample analysis device further comprises a reagent preparation gripper (7-50), a sample processing gripper (7-20) and an analysis gripper (7-30), wherein
the reagent preparation gripper (7-50) is capable of moving in a first direction and/or a second direction in the first independent space (1-21), the second direction being perpendicular to the first direction on a horizontal plane, the reagent preparation gripper (7-50) having a first gripper structure (7-52) capable of moving in a vertical direction, and the first gripper structure (7-52) being configured to grip the first container (11-20) or the second container (11-30) located in the first independent space (1-21);
the sample processing gripper (7-20) is capable of moving in the first direction in the second independent space (1-22), the sample processing gripper (7-20) having a second gripper structure (7-21) capable of moving in the vertical direction, and the second gripper structure (7-21) being configured to grip the first container (11-20) or the second container (11-30) located in the second independent space (1-22); and
the analysis gripper (7-30) being capable of moving in the second direction in the third independent space (1-23), the analysis gripper (7-30) having a third gripper structure (7-31) capable of moving in the vertical direction, and the third gripper structure (7-31) being configured to grip the second container (11-30) located in the third independent space (1-23).

15. The sample analysis device according to claim 2, wherein the sample analysis device further comprises a sample processing gripper (7-20), the sample processing gripper (7-20) being provided with a second gripper structure (7-21), and the sample processing apparatus (1.2) further comprising a film sealing mechanism (33-30) arranged in the second independent space (1-22), wherein
the film sealing mechanism (33-30) comprises:
a third rack (33-10);
a hot-pressing mechanism (33-20) movably arranged on the third rack (33-10) in a vertical direction, the second gripper structure (7-21) being arranged on a side of the hot-pressing mechanism (33-20); and
a bearing mechanism (33-40), the bearing mechanism (33-40) comprising a first placement area and a second placement area which are adjacently arranged, the bearing mechanism (33-40) being movably arranged between the hot-pressing mechanism (33-20) and the second gripper structure (7-21), so that the bearing mechanism (33-40) has a hot-pressing position for fitting the first placement area and the hot-pressing mechanism (33-20), a clamping position for fitting the second placement area and the second gripper structure (7-21), and an assembly position for fitting the first placement area and the second gripper structure (7-21), wherein
the first placement area is configured to place the second container (11-30), the second container (11-30) being configured to place the mixture to be detected, and the second placement area being configured to place a cover body (11-40);
and/or
wherein the analysis apparatus (1.3) comprises a plurality of amplification mechanisms (4-01) sequentially arranged in a second direction,
each of the plurality of amplification mechanisms (4-01) comprising:
a second rack body (4-100), the second rack body (4-100) being provided with a detection window (4-110);
a detection apparatus (4-200), arranged on the second rack body (4-100) and located at the detection window (4-110);
a reagent loading apparatus (4-300), the reagent loading apparatus (4-300) comprising a bracket (4-310), the second container (11-30) being detachably arranged on the bracket (4-310), the bracket (4-310) being movably arranged on the second rack body (4-100) in the first direction, the second direction being perpendicular to the first direction on a horizontal plane, and the second container (11-30) moving along with the bracket (4-310) and having a storage position located below the detection window (4-110) and a loading position for moving out of the second rack body (4-100); and
a temperature control assembly (4-410), movably arranged on the second rack body (4-100) in a vertical direction and being capable of driving the second container (11-30) to ascend and descend, so that the second container (11-30) is lifted to a detection position in butt joint fit with the detection apparatus (4-200).
